# EUROPEAN PATENT SPECIFICATION

(11) **EP 1 791 860 B1**
(45) Date of publication and mention of the grant of the patent: **23.04.2008**
(21) Application number: 05797390.1
(22) Date of filing: 20.09.2005
(51) Int. Cl.: C07K 14/195, C07K 14/315, A61K 39/02, A61K 39/09, C07K 1/20

(54) **PURIFICATION PROCESS FOR BACTERIAL CYTOLYSIN**
REINIGUNGSVERFAHREN FÜR BAKTERIELLES CYTOLYSIN
PROCEDE DE PURIFICATION DE LA CYTOLYSINE BACTERIENNE

(30) Priority: 22.09.2004 GB 0421083
(43) Date of publication of application: 06.06.2007
(73) Proprietor: GlaxoSmithKline Biologicals S.A., 1330 Rixensart (BE)
(72) Inventor: BIEMANS, Ralph L., GlaxoSmithKline Biologicals sa, 1330 Rixensart (BE); GORAJ, Carine, GlaxoSmithKline Biologicals s.a., 1330 Rixensart (BE); MERTENS, Emmanuel, GlaxoSmithKline Biologicals sa, 1330 Rixensart (BE); VANDERCAMMEN, A., GlaxoSmithKline Biologicals sa, 1330 Rixensart (BE)
(74) Representative: Johnston, Caroline Louise
(86) International application number: PCT/EP2005/010258
(87) International publication number: WO 2006/032499

(56) References cited:
- WO-A-90/06951
- WO-A-20/04081515
- US-A- 5 258 504
- MITCHELL T J ET AL: "EXPRESSION OF THE PNEUMOLYSIN GENE IN ESCHERICHIA-COLI RAPID PURIFICATION AND BIOLOGICAL PROPERTIES" BIOCHIMICA ET BIOPHYSICA ACTA . GENE STRUCTURE AND EXPRESSION, ELSEVIER, AMSTERDAM, NL, vol. 1007, no. 1, 1989, pages 67-72, XP002285394 ISSN: 0167-4781
- JOHNSON M K ET AL: "THE HYDROPHOBIC CHARACTER OF THIOL ACTIVATED CYTO LYSINS" BIOCHEMICAL JOURNAL, vol. 207, no. 3, 1982, pages 557-560, XP002357594 ISSN: 0264-6021
- QUEIROZ J A ET AL: "Hydrophobic interaction chromatography of proteins" JOURNAL OF BIOTECHNOLOGY, ELSEVIER SCIENCE PUBLISHERS, AMSTERDAM, NL, vol. 87, no. 2, 4 May 2001 (2001-05-04), pages 143-159, XP004232077 ISSN: 0168-1656
- PATON J C ET AL: "Purification and immunogenicity of genetically obtained pneumolysin toxoids and their conjugation to Streptococcus pneumoniae type 19F polysaccharide." INFECTION AND IMMUNITY. JUL 1991, vol. 59, no. 7, July 1991 (1991-07), pages 2297-2304, XP002357595 ISSN: 0019-9567

## Description

### Technical field

The present invention relates to the field of bacterial cytolysin purification and particularly to a method of purification of pneumolysin. Pneumolysin is a protein from Streptococcus pneumoniae with good antigenic properties which is suitable as a vaccine component against *S. pneumoniae* infection or otitis media. The method of the invention describes an unusual and advantageous step of purifying pneumolysin in a single chromatographic step by binding it to a hydrophobic interaction column in the presence of detergent and high salt. The process advantageously makes use of the property of bacterial cytolysins of having a high affinity for aromatic compounds resembling cholesterol, particularly when in an aggregated condition and hence will be generally applicable for the purification of members of this family of toxins. A further advantageous aspect of the invention is the preparation of the sample to be loaded onto the column by a process of mechanical breakage and prefiltration.

Thiol activated cytolysins form a prominent group of bacterial toxins of which steptolysin O is the prototype (Billington et al FEMS Microbiol. Lett. (2000),182; 197-205). These toxins are lytic for eukaryotic cells by the formation of pores in the cell membrane. Oxidising agents adversely affect their cytolytic activity whereas reducing agents can restore activity. Members of this group show 30-60% similarity in primary amino acid sequence and contain an almost invariant undecapeptide sequence near the C-terminus. Cholesterol is the major target cell receptor for these toxins. The cytolysins bind to cholesterol containing membranes and oligomerise to form transmembrane pores up to 30nm in diameter and composed of 40-80 monomer subunits. The binding of membrane cholesterol induces a conformational change in the toxin monomer driving the subsequent events of oligimerisation, membrane insertion and pore formation.

*Streptococcus pneumoniae* is the causative agent of several human diseases including pneumonia, bacteremia, meningitis, otitis media and sinusitis. Sometimes these diseases can lead to fatalities despite the availability of antibiotics. The emergence of antibiotic resistant strains of *S. pneumoniae* has aggravated the problems caused by this pathogen. In this context, it is important for effective vaccines against *S. pneumoniae* to be developed.

Polyvalent pneumococcal vaccines containing purified capsular polysaccharides have been available for several years. Their application is limited by poor immunogenicity particularly in high-risk groups including infants, the elderly and those with sickle-cell anaemia, multiple myeloma, cirrhosis or alcoholism. They also provide serotype specific protection and only 23 out of 90 known serotypes are covered by existing formulations. This will give protection against 90% of serotypes found in the US population but against only approximately 70% of serotypes found in Asian populations. Recently a conjugated seven-valent vaccine has become available, which similarly has problems protecting against all pneumococcal strains.

Pneumolysin (Ply) is a 53kDa thiol-activated cytolysin found in all strains of *S. pneumoniae,* which is released on autolysis and contributes to the pathogenesis of *S. pneumoniae.* It is highly conserved with only a few amino acid substitutions occurring between the Ply proteins of different serotypes. Pneumolysin's high degree of conservation and its immunogenicity make it a potential candidate as a vaccine component. However, wild-type Ply is unsuitable for incorporation into vaccines for use in humans because of its toxicity. Ply causes damage to cell membranes by interacting with membrane-bound cholesterol and oligomerising to form pores in the membrane. A conserved cysteine-containing motif found near the C-terminus has been implicated in the lytic activity. Mutations of Ply have been suggested to lower this toxicity (WO90/06951, WO99/03884).

A two step method for the purification of pneumolysin has been described by Lock et al (Microbial Pathogenesis (1996) 21; 71-83). Recombinant pneumolysin is purified from an *E. coli* culture using a combination of ion-exchange and gel filtration chromatography. The method involves the steps of preparing an extract and passing it down a DEAE-Sepharose column followed by a Sephacryl S200-HR column. This method could be used to purify recombinant or native pneumolysin.

Kuo et al describe a method of purifying recombinant GST-pneumolysin fusion protein (Infection and Immunity (1995) 63; 2706-2713). The fusion protein is expressed in an E. coli culture and a cell lysate is loaded onto a glutathione agarose gel. The fusion protein is eluted with glutathione and thrombin can be used to cleave the fusion protein. The proteins were passed over a glutathione-agarose column again to remove GST. The affinity purified pneumolysin was further purified using a hydroxylapatite column.

Mitchell et al (BBA (1989) 1007; 67-72) describe a method of purifying pneumolysin using hydrophobic interaction chromatography. Under the conditions that they use (250mM NaCI), the pneumolysin failed to bind tightly to the column although its progress was retarded and the pneumolysin eluted as a broad peak. Additional steps of determining which fractions contained pure pneumolysin, concentrating the positive fractions, reloading onto the column and eluting with a small volume of water were needed to overcome the problem of the pneumolysin not binding tightly to the column material.

There remains a continuing need for improved vaccines against *S. pneumoniae*. The incorporation of a Ply component has promise although the toxicity of the protein remains a problem. The development of a rapid and effective procedure for the bulk purification of pneumolysin is also required. Methods described previously involve the use of multiple purification steps with intervening assay and concentration steps. The present invention provides a more efficient purification method which advantageously uses a single chromatography step, which is capable of being used to purify large batches of pneumolysin.

### Description of figures

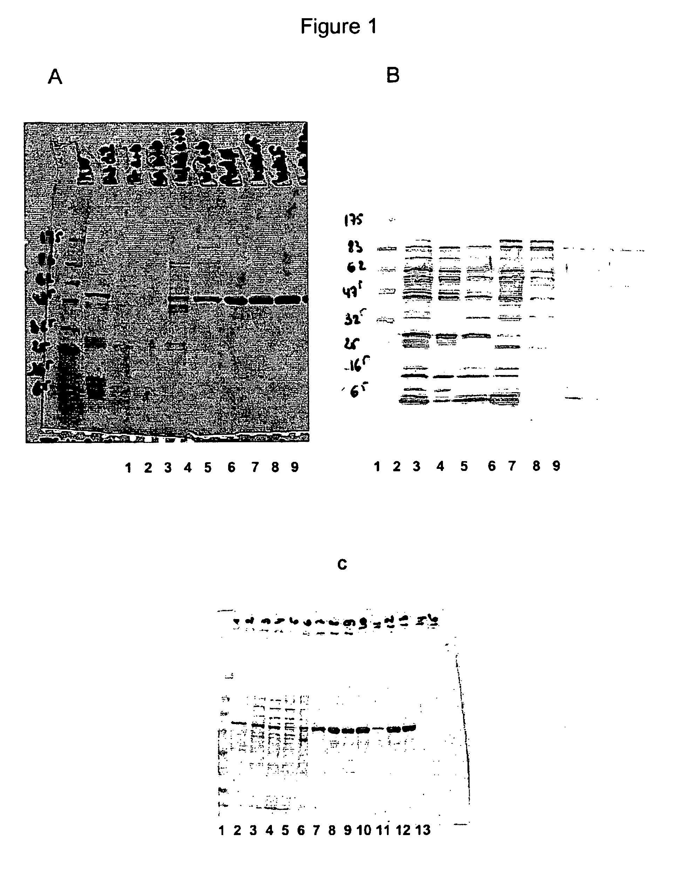

Figure 1 - SDS-PAGE gels showing the purification of pneumolysin.
Panels A and B show purification of pneumolysin following the method of example 1. Panel A shows the gel after coomassie blue staining. Panel B shows the gel after a Western blotting procedure using anti- E.coli antibodies to probe for contaminating proteins.

The following samples were run on SDS-PAGE gels:- lane 1 - molecular weight standards, lane 2 - supernatant of cell extract, lane 3 - phenyl-sepharose flow through, lane 4 phenyl sepharose first wash, lane 5 - phenyl-sepharose second wash, lane 6 phenyl-sepharose wash with 0.5M NaCl, lane 7 Phenyl-sepharose elution with low salt buffer, lane 8 pneumolysin after denaturation/refolding steps, lane 9 - pneumolysin after sterilizing filtration.

Panel C shows a commassie stained gel showing purification of penumolysin following the method of example 2.

The following samples were run on SDS-PAGE gels:- lane 1 - molecular weight standards, lane 2 - fermentor broth Rannie treated, lane 3 - broth filter clarified, lane 4 filter clarified sample diluted to OD60, lane 5 - phenyl-sepharose flow through, lane 6 phenyl-sepharose wash, lane 7 Phenyl-sepharose wash with 0.5M NaCl, lane 8 Phenyl-sepharose elution with low salt buffer, lane 9 - pneumolysin after sterilizing filtration.

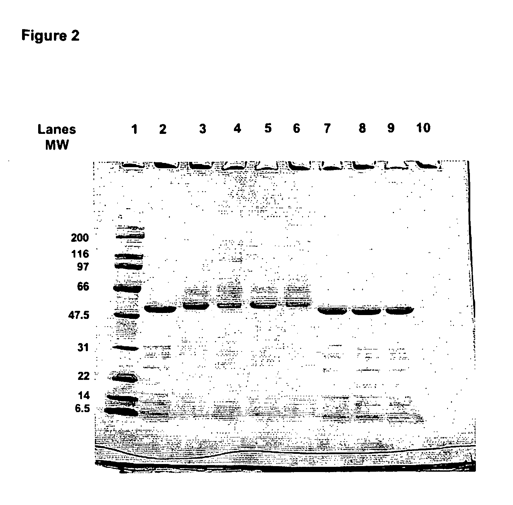

Figure 2 - SDS-PAGE analysis of GMBS (N-(γ-maleimidobutyryloxy)succinimide ester) modified pneumolysin - coomassie blue stained.

The following samples were run on an SDS-PAGE gel:- lane 1 - molecular weight standards, lane 2 - unmodified pneumolysin, lane 3 - PLY treated with GMBS at a molar ratio of GMBS/lysine of 4/1, lane 4 - PLY treated with GMBS at a molar ratio of GMBS/lysine of 4/1 and incubated for 7 days at 37°C, lane 5 - PLY treated with GMBS at a molar ratio of GMBS/lysine of 8/1, lane 6 - PLY treated with GMBS at a molar ratio of GMBS/lysine of 8/1 after incubation for 7 days at 37°C, lane 7 - PLY treated with Sulfo-NHS acetate at a molar ratio of NHS/lysine of 10/1, lane 8 - PLY treated with NEM, lane 9 - PLY treated with NEM after 7 days incubation at 37 °C.

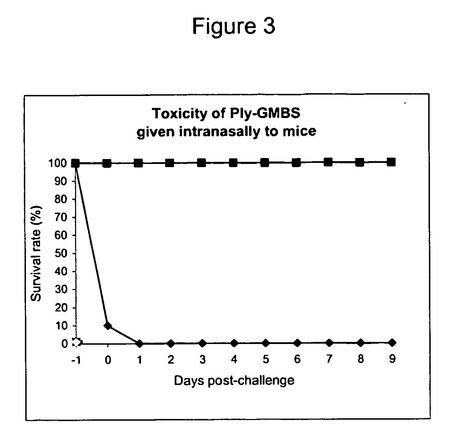

Figure 3 - Toxicity of GMBS treated pneumolysin given intranasally to mice. The line marked with diamonds indicates survival rate for mice challenged with 2µg native pneumolysin. The line marked with squares indicates the survival rate for mice challenged with 10µg GMBS treated pneumolysin.

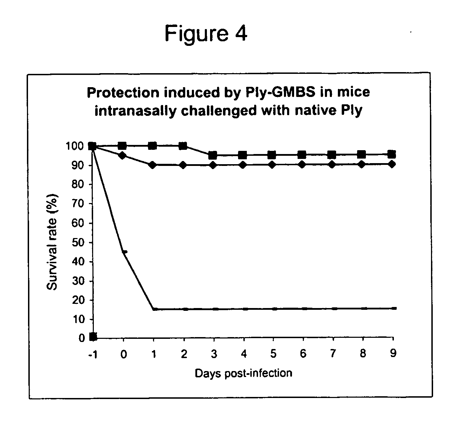

Figure 4 - Protection induced by GMBS treated pneumolysin in mice challenged intranasally with native pneumolysin. The line marked with rectangles shows survival rate in mice inoculated with adjuvant alone. The line marked with diamonds indicates the survival rate for mice inoculated with native pneumolysin. The line marked with squares indicates the survival rate for mice inoculated with GMBS treated pneumolysin.

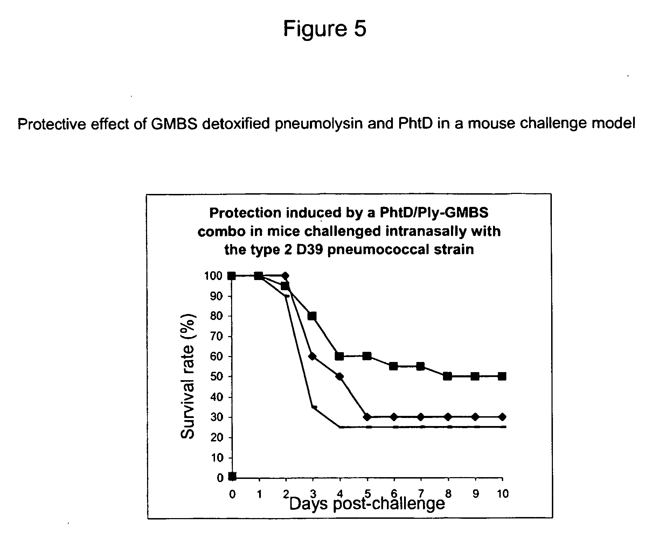

Figure 5 - Protection induced by Inoculation with PhtD and GMBS treated pneumolysin in mice challenged intranasally with type 2 D39 pneumococcal strain. The line marked with rectangles represents survival rate for mice inoculated with adjuvant alone. The line marked with diamonds represents the survival rate for mice inoculated with PhtD. The line marked with squares represents the survival rate for mice inoculated with PhtD and GMBS treated pneumolysin.

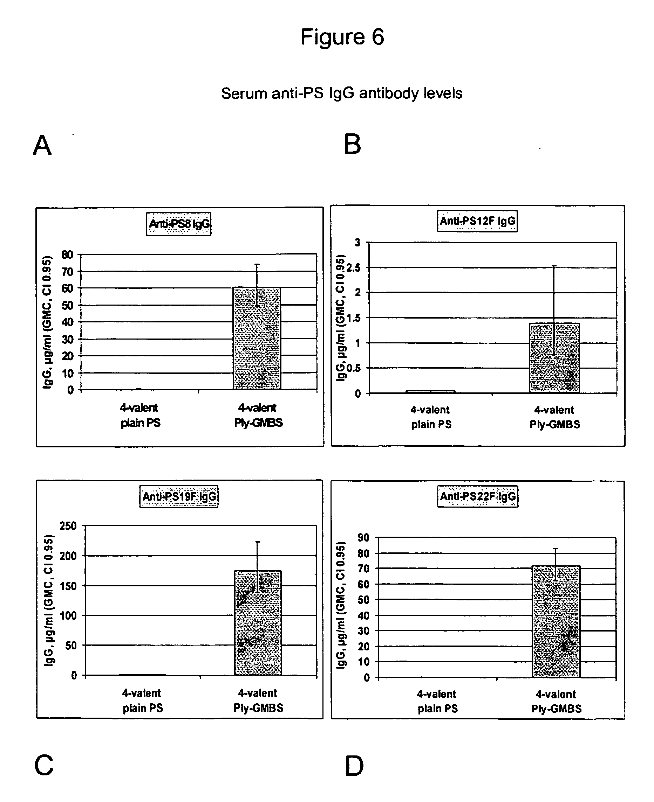

Figure 6 - ELISA results showing anti-polysaccharide IgG antibody levels following inoculation with 4-valent plain polysaccharide or 4-valent polysaccharides conjugated to GMBS detoxified pneumolysin. Panel A shows levels of anti-PS8 IgG. Panel B shows levels of anti-PS12F IgG. Panel C shows levels of anti-PS19F IgG. Panel B shows levels of anti-PS22F IgG.

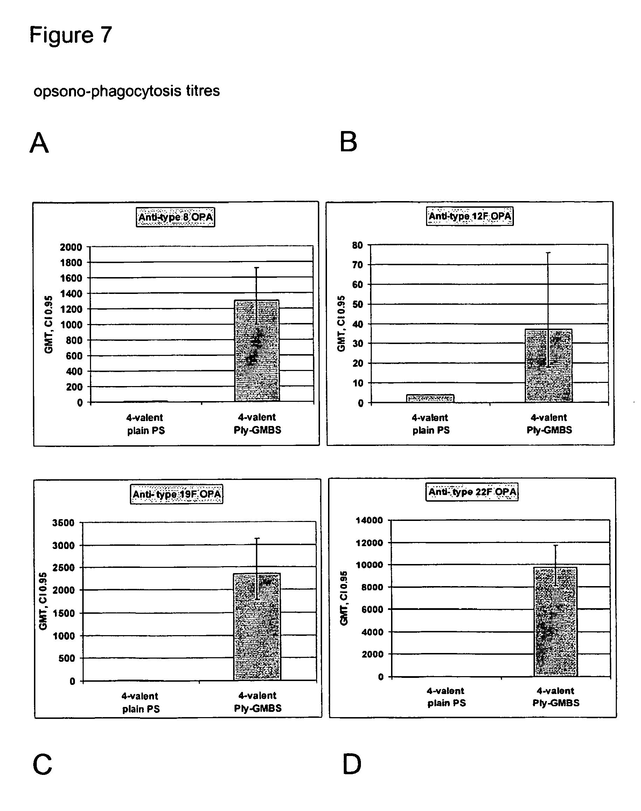

Figure 7 - Opsono-phagocytosis GMTs following inoculation with 4-valent plain polysaccharide or 4-valent polysaccharides conjugated to GMBS detoxified pneumolysin. Panel A shows results of an anti-type 8 opsono-phagocytosis assay. Panel B shows results of an anti-type 12F opsono-phagocytosis assay. Panel C shows results of an anti-type 19F opsonophagocytosis assay. Panel B shows results of an anti-type 22F opsonophagocytosis assay.

### Detailed description

### Processes

The process of the invention is a method for purifying a bacterial cytolysin such as pneumolysin. A cytolysin, for instance pneumolysin, is purified using a single column chromatography step. The protein is bound in an aggregated form to a hydrophobic interaction column in the presence of detergent and salt. Few proteins bind to the column under these conditions allowing purification of a cytolysin in a single step. The process of the invention is particularly applicable to bulk purification of a cytolysin since in a preferred embodiment of the invention, a centrifuge is not used during the preparation of a lysate to be loaded onto the column. The use of a centrifuge is often a limiting step in the production process. In further preferred embodiments of the invention, the denaturation/renaturation step is carried out on concentrations of cytolysin of typically greater than 100µg/ml.

For the purposes of the invention a soluble aggregate of a cytolysin, preferably pneumolysin is an aggregated form of the cytolysin that remains in the supernatant after centrifugation at 30,000g for 20 minutes. The soluble aggregate is retained on hydrophobic interaction chromatography material, preferably phenyl-Sepharose, in the presence of detergent and high salt, preferably 1M. Optionally, the soluble aggregate is colloidal.

Salt concentration includes the concentration of all salts, including buffering salts, present in a solution or suspension.

For the purposes of the invention, low salt conditions have a conductivity of below 5mS/cm, preferably 1-2mS/cm. High salt conditions have a conductivity of greater than 30mS/cm, preferably greater then 50mS/cm, more preferably of 60-80 mS/cm.

The cytolysin, preferably pneumolysin is bound to the column as a soluble aggregate. It is unusual to load aggregates onto a column for various reasons including filters or columns clogging and loss of material. However, by using a detergent, preferably at an alkaline pH, that reduces the size of the aggregates to form a soluble aggregate, it is found that these aggregates bind tightly to the column under detergent conditions but may be eluted at a purity of at least 50%, 60%, 70%, 80%, preferably 90%, 95%, more preferably 97%, 98% or 99% as assessed by SDS-PAGE analysis without adversely affecting the column filters. The process preferably gives a yield of at least 100, 200, 500, 700, more preferably 1000, 1500, 1700 or 1900mg of cytolysin, preferably pneumolysin per litre of fermentation. Preferably at least 1%, 2%, 5%, 7%, 9% or 10% of the protein from the fermentation culture is recovered as purified cytolysin, preferably pneumolysin.

The process exploits the ability of cytolysins such as pneumolysin to bind to cholesterol and other aromatic compounds. This binding is particularly tight when the cytolysin is aggregated, allowing the cytolysin to bind in the presence of detergent. The process can be extended to other members of the cytolysin family since all members share the ability to bind to aromatic compounds and form pores. In fact the method could be used to purify other families of protein that bind to cholesterol or other aromatic compounds and/or form pores, preferably both.

Accordingly, in a first embodiment, a process for bacterial cytolysin purification is provided comprising the steps of:
a) growing a culture of cells expressing bacterial cytolysin;
b) mechanically breaking the culture of cells to form an extract;
c) prefiltering the extract;
d) binding soluble aggregated bacterial cytolysin contained in the extract in the presence of detergent (preferably aliphatic detergent) to a hydrophobic interaction chromatography material under high salt (preferably 0.5-2M salt) conditions;
e) eluting bacterial cytolysin in the presence of detergent (preferably aliphatic detergent) under low salt (preferably 0-0.2M salt) conditions.

In a second embodiment a process for bacterial cytolysin purification is provided comprising the steps of:
a) growing a culture of cells expressing bacterial cytolysin;
b) mechanically breaking the culture of cells to form an extract:
c) prefiltering the extract;
d) binding bacterial cytolysin contained in the extract to hydrophobic interaction chromatography material in the presence of a solution containing 0.5-2M or 0.7-1.5M preferably 0.8-1.2M salt and 0.1%-1.5% preferably 0.5-1.2% detergent;
e) eluting bacterial cytolysin using a low salt (preferably 0-0.2M salt) solution containing 0.1-1.5%, preferably 0.5-1.2% detergent.

In either of the above embodiments, the process of the invention preferably comprises the further steps of:
f) removing detergent from the bacterial cytolysin
g) solubilising the bacterial cytolysin by addition of a denaturant;
h) removing the denaturant from the bacterial cytolysin.

The following description of the invention applies to either of the embodiments listed above.

The process of the invention can be advantageously used to purify pneumococcal pneumolysin. Other cytolysins that can be purified by the method of the invention include pyolysin from *A. pyogenes,* cereolysin from *B*. *cereus,* thuringiolysin O from *B*. *thuringiensis,* laterosporolysin from *B*. *latersporus,* bifermentolysin from *C. bifermentans*, botukinolysin from *C. botulinum,* chauveolysin from *C. chauvoel*, histolyticolysin from *C. histolyticum,* oedematolysin from *C. novyi* type A, perfringolysin O from *C. perfringens*, septicolysin O from *C. septicum*, sordellilysin from *C. sordellii*, tetanolysin from *C. tetani*, ivanolysin O from *L. ivanovi*, listeriolysin O from *L. monocytogenes*, seeligerilysin O from *L*. *seeligeri,* alveolysin from *P. alvei*, streptolysin O from *S. pyogenes*, *S. canis* or *S. equisimilis*, intermedilysin from *S. intermedius*, suilysin from S. suis or pneumolysin from *S. pneumoniae* which may be of wild type or may be a genetically modified toxins with lower levels of toxicity such as PdA and PdB (WO90/06951, WO99/03884).

By pneumolysin or Ply it is meant: native pneumolysin from pneumococcus or recombinant pneumolysin, wild-type pneumolysin or mutants of pneumolysin (e.g. those described in WO90/06951 and WO99/03884). Optionally, pneumolysin can also mean any fragment of pneumolysin or any variant of pneumolysin which shares at least 70, 80, 90 or 95% amino acid sequence identity with a wild-type pneumolysin sequence (as disclosed in Walker et al 1987, Infect. Immun. 55; 1184-9 or Mitchell et al 1990, Nucleic Acids Res. 18; 4010), which still retains the ability to be purified by the methods of the invention, as easily determined by a skilled person.

In preferred embodiments of the invention, the same detergent is present in steps b) and d), b) and e), d) and e), more preferably in steps b), d) and e). Preferably the same detergent is present is step c) as is present in steps b) and d), b) and e),or d) and e), more preferably in steps b), d) and e). The detergent is preferably present at a concentration of 0.1%-1.5% (w/v), more preferably 0.5%-1.2% (w/v) or around 1% (w/v). For the purposes of the invention, an aliphatic detergent is defined as a substantially aliphatic detergent with insufficient aromatic character to prevent binding of cytolysin to the column in step d). Preferably, the detergent will have one or less aromatic ring, most preferably it has no aromatic ring. During step b), it is advantageous for the detergent to break up larger aggregates of cytolysin into smaller aggregates which make a soluble aggregate. During steps d) and e), the detergent advantageously retains the soluble aggregated state of the cytolysin, allowing it to bind to the column in high salt conditions with high affinity.

The cytolysin, preferably pneumolysin is expressed in a culture of bacterial cells, preferably *S. pneumoniae, E. coli* or alternatively in yeast cells, insect cells, mammalian cells or any other expression system suitable for its expression. In expression systems that produce high yields of pneumolysin, the pneumolysin often becomes aggregated of its own accord and the process of the invention is ideal for its purification. Preferably pneumolysin is expressed at high yields so that it makes up more than 2, 3, 4, 5, 7 or 10% of total protein in the expression system. Preferably the pneumolysin is in aggregated form and/or mostly devoid of haemolytic activity. For example, expression in *E*.*coli* in a fermentor under a phage λ promoter or other promoters that allow high expression are well known to the person skilled in the art.

Preferably, the cytolysin is extracted from the expression system as an aggregate. Alternatively, a lower yield expression system may provide soluble cytolysin. In this case, the extract containing cytolysin, preferably pneumolysin is adjusted to a pH below 7.5 which allows the cytolysin to aggregate over a period of at least 8 hours, preferably at least 24 hours.

The mechanical breakage in step b) preferably involves one, two, three, four, five, six, seven, eight, nine, ten or more steps of mechanically breaking the cells (optionally by Rannie passes) and/or treating the cells with detergent. If made with a high yield method, the pneumolysin remains in the form of aggregates but the aggregates should be small enough so that they remain in the supernatant after centrifugation of the sample under conditions necessary for pelleting insoluble cellular debris. Preferably the detergent used in the invention is an aliphatic detergent which does not contain aromatic rings, preferably an ionic detergent, more preferably a cationic or anionic detergent and most preferably, the detergent is sodium lauroyl sarcosinate. Preferred detergents are able to solubilise pneumolysin whilst leaving it in the form of small aggregates that bind to the hydrophobic interaction column without causing blockage of filters attached to the column. Preferred detergents are able to reduce the size of pneumolysin aggregates, allowing the pneumolysin aggregates to be sufficiently small so that they remain in the supernatant after centrifugation of the sample at 30,000g for 20 minutes. Such soluble aggregates are purifiable as such on the hydrophobic interaction column. The detergent is present at a concentration of between 0.1% and 1.5%, preferably 0.5% and 1.2% (w/v), more preferably around 1%. Preferably, the detergent is dialysable.

Following mechanical and/or detergent disruption of the culture in step b), the process of the invention includes a prefiltration step c) for removing larger aggregated material which could block the column in subsequent steps. Preferably the prefiltration step uses a filter with a pore size of 0.4µm, 0.65µm, 1.2µm, 2.45µm or 5µm. Preferably the filter has a pore size of 0.45-2.5µm or 0.6 - 1.2µm. Preferably the mechanical breakage and prefiltration steps allow the process of the invention to avoid the use of centrifugation.

Optionally, the process of the invention contains a preincubation step where the culture of cells is incubated with detergent before mechanically breaking the culture of cells. The preincubation step involves addition of the detergents described above for at least 1, 5, 10, 20, 30, 60 or 120 minutes before mechanical breakage of the culture of cells.

Preferably, step b) and/or c) is carried out at alkaline pH, preferably a pH of 8-10, 8.5-9.5 or around 9. Preferably steps f), g) and h) are carried out at alkaline pH, preferably a pH of 8-10, 8.5-9.5 or around 9. Preferably step d) and/or e) is carried out at neutral pH, preferably a pH of 6-8, 6.5-7.5 or around 7. Preferably step b) and/or c) is carried out at a salt concentration of 0-0.1M, more preferably 10-50mM or 20-30mM. Preferably steps f), g) and h) are carried out at a salt concentration of 0-0.1M, more preferably 10-50mM or 20-30mM.

The process of the invention uses hydrophobic interaction chromatography to purify pneumolysin in a single step. The column material used in step d) preferably contains aromatic groups, preferably phenyl groups and more preferably is phenyl-sepharose.

The solution used in step d) and/or step e) during loading and elution of the column comprises an ionic detergent, preferably a cationic or anionic detergent, preferably a detergent which is soluble at salt concentrations above 0.5M, most preferably the detergent is sodium lauroyl sarcosinate. The detergent used is one which will reduce the size of cytolysin, preferably pneumolysin, aggregates, allowing the cytolysin to be present in the sample as a soluble aggregate so that it will bind to the hydrophobic interaction column material without being irreversibly stuck on the column. The detergent is present at a concentration of preferably between 0.1% and 1.5%, preferably 0.5% and 1.2% (w/v), more preferably between 0.75% and 1.2%, most preferably around 1%.

The solution used in step d) and/or e) contains a salt, preferably a salt selected from the group consisting of sodium chloride, magnesium chloride, ammonium chloride, sodium sulphate, magnesium sulphate, ammonium sulphate, sodium phosphate, magnesium phosphate, ammonium phosphate and is preferably buffered at pH 6-8, preferably around pH 7. Any buffer capable of maintaining the pH at a set value between pH 6 and 9 may be used.

The solution used to bind pneumolysin to the column in the process of the invention contains a high salt concentration, preferably 0.6 - 2M, more preferably around 1M. The salt concentration is chosen such that pneumolysin is in a soluble aggregated form and is capable of binding to the hydrophobic chromatography material.

A single column purification is often capable of purifying a cytolysin, preferably pneumolysin to at least 90, 92, 94, 96, 98, or 99% pure. However, in some embodiments of the invention, where a small column size is used or where a large quantity of cytolysin, preferably pneumolysin is loaded onto the column, an extra 1, 2, 3 or more passages down a similar column under the conditions described above, may be used to achieve higher levels of purity.

Optionally, step d) can contain an extra step of washing the column in intermediate salt conditions of around 0.5M salt or a salt concentration capable of removing any poorly binding impurities.

The process of the invention uses a decreasing salt gradient to elute pneumolysin from the column. Preferably the low salt solution used to make the salt gradient in step e) contains between 0 - 0.1M salt, more preferably 0-40mM salt. Alternatively, step wise elution may be used with the low salt buffer used in step e) containing between 0 - 0.2M salt, more preferably 0-40mM salt.

Optional steps may be added to the process of the invention if it is preferred to denature the pneumolysin and subsequently refold it by removal of the denaturant. These optional steps ensure that pure cytolysin, preferably pneumolysin, with a native structure and/or biological activity (e.g. haemolysis of erythrocytes) is obtained. The first optional step f) involves the removal of detergent by diafiltration, dialysis or dilution. This step preferably involves diafiltration/dialysis against a buffer of pH 8-10, preferably around 9, more preferably the buffer is one able to buffer at alkaline pH values, most preferably the buffer is diethanolamine (DEA). The solution is preferably of low ionic strength, preferably 10-50mM, most preferably around 25 mM. Diafiltration or dialysis is preferably carried out at 4°C but is alternatively carried out at room temperature.

In a second optional step, cytolysin, preferably pneumolysin is denatured and solubilised by addition of a denaturant. Preferably the denaturant used in step g) is guanidine hydrochloride, more preferably 5-8M guanidine hydrochloride, most preferably around 6M guanidine hydrochloride. The pneumolysin is incubated with guanidine hydrochloride for at least 10 minutes, preferably for at least 1 hour, more preferably for about one hour.

The cytolysin, preferably pneumolysin is preferably then contacted with 5-9M urea, preferably around 8M urea during step g). This is achieved by diafiltration or dialysis of the cytolysin, preferably pneumolysin against urea. Preferably, the same buffer and pH are maintained during the exchange of denaturant. Preferably, a reducing agent (DTT, 2-mercaptoethanol or glutathione is added during the exchange of denaturant.

Preferably step g) involves contacting cytolysin, preferably pneumolysin with 5-8M guanidine hydrochloride followed by exchanging the guanidine hydrochloride for 5-9M urea.

In order to prevent inappropriate disulphide bonds forming while the cytolysin, preferably pneumolysin is denatured, it is advantageous to ensure that a reducing agent is present during at least part of steps g) and h). A preferred reducing agent is 0.1-10mM DTT, preferably around 1mM DTT. Alternatively glutathione or 2-mercaptoethanol is used. Preferred concentration of glutathione are 1-20 mM or 5-15mM, more preferably 5-10mM.

Optional step h) involves removal of the denaturant in order to refold cytolysin, preferably pneumolysin, preferably by diafiltration or dialysis against a low salt buffer of pH 6-10, preferably around pH 9. Preferably the denaturant is removed in a linear fashion rather than the logerithmic fashion normally associated with dialysis or diafiltration. That is, the denaturant is removed from the cytolysin at a similar or constant rate throughout the dialysis or diafiltration step or the denaturant is removed at a slower rate at the beginning of the diafiltration step than it is at the end. This is in contrast to most methods where the denaturant is removed at a faster rate towards the beginning of the process. This is achieved either by carrying out diafiltration at progressively higher recirculation flow rates or by dialysing against solutions containing progressively less denaturant. For instance, diafiltration may be carried out at rates between 50ml/hour and 500ml/hour and the speed of diafiltration can be increased on 1, 2, 3, 4, 5, 6, 7, 8, 9, 10 or more occasions. Preferably cytolysin, preferably pneumolysin concentration is maintained at at least 100µg/ml, preferably between 100 µg/ml and 1000µg/ml, more preferably at around 500µlg/ml. Optionally, diafiltration or dialysis is against a buffer containing propylene glycol at between 10 and 30%, preferably at around 15%. Preferably a reducing agent as described above is maintained during step h). Diafiltration or dialysis is preferably carried out at 4°C but is alternatively carried out at room temperature. This refolding method is applicable to other proteins.

A further optional step i) involves the removal of the reducing agent after cytolysin, preferably pneumolysin has refolded. This is preferably achieved by diafiltration or dialysis against a low salt buffer of pH 7-10, preferably around pH 9. Optionally, diafiltration or dialysis is against a buffer containing propylene glycol at between 10 and 30%, preferably at around 15%. Diafiltration or dialysis is preferably carried out at 4°C but is alternatively carried out at room temperature.

In preferred methods of the invention, the cytolysin, preferably pneumolysin is refolded so that its haemolytic activity is restored to above 25%, 50%, 75% most preferably to above 90% of that of the same quantity of properly folded protein. For the purposes of the invention, 'folded' protein is a protein having the tertiary structure of the protein made by a non-denaturing process. In the case of wild type pneumolysin, the expected haemolytic activity of refolded pneumolysin would be 500,000-1,000,000 haemolytic units/mg pneumolysin. In the case of point mutated pneumolysin with a lower haemolytic activity, the haemolytic activity of the refolded pneumolysin would be correspondingly lower.

### Detoxification of a toxin

The cytolysin purified by the method of the invention, preferably pneumolysin may be subjected to a further optional step of detoxification by chemical treatment. This additional step is particularly advantageous if the cytolysin, preferably pneumolysin is to be administered to an animal or a human. Wild type pneumolysin is highly toxic. Several mutated pneumolysin proteins have been isolated that have reduced toxicity, yet these still retain residual toxicity that may be problematic when the pneumolysin is administered internally (WO99/03884, WO90/06951). Alternatively it can be detoxified by conjugation to saccharides (WO96/05859).

The process of the invention may detoxify either wild type or mutated cytolysin, for example pneumolysin by chemical treatment. Preferred embodiments use a crosslinking agent, more preferably containing one or more chemicals selected from the group consisting of formaldehyde, glutaraldehyde and a cross-linking reagent containing an N-hydroxysuccinomido ester and/or a maleimide group (e.g. GMBS).

The detoxification processes themselves are an aspect of the invention and can be used to detoxify bacterial toxins, preferably pneumolysin prepared by other methods.

In one embodiment, the detoxification method of the invention describes the detoxification of a bacterial toxin comprising treating the toxin with a chemical compound, preferably a crosslinking reagent that is reactive, preferably preferentially reactive, most preferably specifically reactive with amine groups, more preferably primary amine groups.

For the purposes of this application, a cross linking reagent is defined as a compound with at least two reactive groups, at least one of which is capable of reacting with at least one group on the bacterial toxin. A further reactive group is able to react with either a group on the bacterial toxin or a separate compound (for instance an amino acid, peptide, polypeptide, sugar or polysaccharide).

Preferably, the chemical compound or the crosslinking reagent is reactive, more preferably preferentially reactive, most preferably specifically reactive with amine and sulfhydryl groups. Preferably, the chemical compound reacts with a primary amine group of lysine, more preferably, the crosslinking reagent reacts with a primary amine group of lysine and the sulfhydryl group of cysteine. This method is particularly advantageous where pneumolysin is detoxified since modification of both cysteine and lysine residues leads to a synergistic decrease in the level of hemolysis compared to the residual hemolysis activity where the cross-linking reagent reacts with only lysine or cysteine.

Thus an alternative embodiment provides a method of detoxifying bacterial toxins comprising modifying a cysteine residue (optionally near the C-terminus of the toxin) involved in the toxic activity of the toxin (preferably the lytic activity) comprising treating the toxin with a cross-linking reagent (preferably a heterobifunctional cross-linking reagent) that cross-links the sulfhydryl groups with another amino acid of the toxin, preferably more than 2, 5, 10, 15, 20, 30, 40 amino acids away from the cysteine in the primary structure. Preferably the other amino acid contains a primary amine group and more preferably the amino acid is lysine.

In some embodiments, over 50%, 60%, 70%, 80%, 90% or 95% of the toxin retains a molecular weight within 5%, 10%, 20%, 30%, 40%, 50%, 60%, 70%, 80% or 90%, more preferably between 1-50%, most preferably between 5-10% of its original molecular weight after the treatment as assessed by SDS-PAGE. Preferably the toxin acquires a slightly higher molecular weight following the detoxification treatment due to several amino acid residues becoming modified by covalently binding to the chemical compound. However the method of the invention preferably does not involve extensive conjugation of the toxin, either by covalently binding it to other toxin molecules so that a toxin with a multimeric quaternary structure is formed, or by covalently binding the toxin to other large proteins, polysaccharides or lipopolysaccharides. Most preferably the methods, proteins or products disclosed in WO96/05859 are not covered by this invention.

The methods of the invention may be used to detoxify bacterial toxins. Preferred toxins include the thiol-activated cytolysins pyolysin from *A. pyogenes*, cereolysin from *B*. cereus, thuringiolysin O from *B. thuringiensis,* laterosporolysin from *B*. *latersporus,* bifermentolysin from *C. bifermentans*, botukinolysin from *C. botulinum,* chauveolysin from *C. chauvoel*, histolyticolysin from *C. histolyticum*, oedematolysin from *C. novyi* type A, perfringolysin O from *C. perfringens,* septicolysin O from *C. septicum,* sordellilysin from *C. sordellii*, tetanolysin from *C. tetani*, ivanolysin O from *L. ivanovi,* listeriolysin O from *L. monocytogenes*, seeligerilysin O from *L*. *seeligeri*, alveolysin from *P. alvei*, streptolysin O from *S. pyogenes*, *S. canis* or *S. equisimilis,* intermedilysin from *S. intermedius*, suilysin from *S. suis* or pneumolysin from *S. pneumoniae* which may be of wild type or may be a genetically modified toxins with lower levels of toxicity such as PdA and PdB (WO90/06951, WO99/03884).

The method may also be used to detoxify the Neisserial toxins FrpA, FrpC (WO92/01460), FrpB (Microbiology 142; 3269-3274, (1996); J. Bacteriol. 181; 2895-2901 (1999)) NM-ADPRT (13^{th} International Pathogenic Neisseria Conference 2002 Masignani et al p135). FrpA and FrpC contain a region which is conserved between these two proteins and a preferred fragment of the toxins would be a polypeptide containing this conserved fragment, preferably comprising amino acids 227-1004 of the sequence of FrpA/C.

The method of the invention may also be used to detoxify Bordetella toxins including adenylate cyclase (CyaA) (Glaser (1988) Mol. Microbiol. 2; 19-30), dermonecrotic toxin (Livey (1984) J. Med, Microbiol. 17; 91-103) and pertussis toxin (PT) (Munoz et al (1981) Infect Immun 33; 820-826). The method of the invention is also useful for detoxifying tetanus toxin (TT) and diphtheria toxin (DT) and toxin from *S. aureus* and *S. epidermidis* including autolysins (AtIE, amidase and glucosaminidase), bone sialo binding protein (HarA) and haemolysin (WO 01/98499; WO 02/59148; WO03/11899 locus 1, 2, 3, 5, 7, 78, 84).

Methods of the invention lead to a reduction of the amount of toxicity and/or haemolytic activity of the toxin of at least 90%, preferably 95%, 96%, 98%, 99%, 99.5%, 99.9% or 99.99%. (Haemolytic activity is measured using the method of Example 3 and toxicity may be measured by the method of Example 5.) Native pneumolysin has a haemolytic activity of 500,000 - 1,000,000 units per mg of pneumolysin. Some point-mutated variants of pneumolysin have reduced toxicity and haemolytic activity. Detoxification of a variant pneumolysin may not be able to achieve as large a percentage decrease in haemolytic activity due to the lower starting point form which haemolytic activity is reduced, however it is envisioned that the majority of the remaining haemolytic activity is removed by the methods of the invention.

The detoxification step of the method of the invention preferably provides a cross-linking reaction which is substantially non-reversible. Reversibility is assessed by monitoring the level of haemolytic activity of the detoxified toxin directly after detoxification and after incubating at a temperature above 25°C, preferably above 30°C, more preferably above 35 °C, most preferably above 37 °C for at least 5, 6, 7, 8, 9 or 10 days. A substantially non-reversible reaction results in substantially non-reversible detoxification and is defined as a reaction where the level of haemolytic activity rises by less than 100%, 50%, 40%, 30%, 20% 10% after incubation at an elevated temperature as described above. Many methods of detoxification, for instance by using formaldehyde treatment, result in detoxification that is not stable but increases in toxicity over time.

In a preferred detoxification step of the method of the invention over 50%, 60%, 70%, 80%, 90%, 95%, or 98% of the toxin retains a monomeric quaternary structure after the cross-linking reaction. Many cross-linking reagents form intermolecular crosslinks (for example formaldehyde and glutaraldehyde). This can effect the immunological properties of the toxin since some epitopes will be hidden within the aggregate. Methods of the invention preferably involve simply modifying amino acid residues, preferably sulfhydryl and/or primary amine groups of amino acids and/or the formation of mainly intramolecular crosslinks. The resultant monomeric quaternary structure allows epitopes to remain exposed on the surface of the toxin.

In a preferred embodiment of the detoxification step, the cross-linking reagent is heterobifunctional. Preferred crosslinking reagents contain an N-hydroxysuccinimide ester group that reacts preferentially, more preferably specifically, with primary amine groups. Preferably the cross-linking reagent contains a maleimide group that reacts preferentially, more preferably specifically, with sulfhydryl groups. At a pH around 7, a maleimide group reacts 1000 fold faster with sulfhydryl groups than it does with amines. Preferably, the cross-linking reagent contains both an N-hydroxysuccinimide ester group and a maleimide group. The crosslinking agent is preferably not cleavable using a reducing agent since this leads to less effective detoxification.

The distance between the reactive groups of the cross-linking reagent is able to effect the efficiency of detoxification. Preferably, the distance between the groups of the crosslinking reagent that are reactive with amine and sulfhydryl groups is between 1.5 and 20 Angstroms, more preferably between 5 and 15 Angstroms and most preferably around 10 Angstroms in the method of the invention. Preferably, amino acid residues on the bacterial toxin are modified by addition of a group that is over 5, 7,10, 12, 15, 18, 20, 50, 100, 500 Angstroms long. Preferably, the modifying group is between 5 and 100 Angstroms, more preferably between 10 and 20 Angstroms in size.

The detoxification step of the method of the invention allows sufficient residues to be modified so that steric interference and/or conformational changes inhibit the function of the bacterial toxin. Preferably at least 5, 7, 10, 12, 14, 15, 20 or 25 amino acid residues of the bacterial toxin are modified. Where unreacted maleimide groups are present on the cross-linking reagent, an Ellman reaction can be used to estimate (indirectly) the number of crosslinker molecules attached to each molecule of toxin (Ellman 1959 Arch. Biochem. Biophys. 82; 70).

Preferred crosslinking reagents are SMPT, Sulfo-LC-SMPT, Sulfo-KMUS, LC-SMCC, KMUA, Sulfo-LC-SPDP, LC-SPDP, SMPB, Sulfo-SMPB, SMPH, Sulfo-SMCC, SMCC, SIAB, Sulfo-SIAB, GMBS (N-(y-maleimidobutyryloxy)succinimide ester), Sulfo-GMBS, MBS, Sulfo-MBS, Sulfo-EMCS, EMCA, EMCS, BMPS, SPDP, SBAP, BMPA, AMAS, SATP and SIA (Pierce).

In a preferred method of the invention the toxin is treated with the chemical compound or crosslinking reagent under pH conditions of between 5.0 and 9.0, preferably 6.5 to 8.0, most preferably 7.0 to 7.8. In treatments where the reaction of a maleimide group to a sulfhydryl group is encouraged, the preferred pH of the reaction is 6.0 and 8.0, more preferably 6.5 and 7.5. The preferred concentration of salts during the reaction is between 100mM and 1M, more preferably 150mM and 500mM, most preferably between 200mM and 300mM. However, the inventors have found that it is sometimes preferable to perform the reaction at low salt concentration where no sodium chloride or other salt is added. Where the reaction is performed at a pH of between 7.6 and 7.8, the reaction can optionally be carried out without the addition of salt. Similarly, the use of higher ratios of GMBS to toxin can be performed without the addition of salt at pH values between 7.0 and 8.0.

Preferably a 50-500, more preferably 130-350 or 350- 900, most preferably around 250 fold molar excess of the chemical compound or crosslinking reagent to each toxin is used. Pneumococcal pneumolysin contains 31 lysine residues. Therefore a 248 fold molar excess of chemical compound or cross-linking reagent over pneumolysin is equivalent to an 8 fold molar excess of chemical compound or cross-linking reagent to each lysine residue. Preferably a 2-20, more preferably a 4-15 or 15-30, most preferably around 8 fold molar ratio of chemical compound or cross-linking reagent to lysine residues is used in methods of the invention.

The treatment with crosslinking reagent proceeds for at least 15 minutes, preferably for at least 30 minutes, most preferably for around one hour at between 4°C and 40 °C, preferably between 15 °C and 25°C, most preferably at room temperature. The method of the invention may further comprise a quenching step using a compound containing a sulfhydryl group, preferably the quenching compound has a molecular weight of over 50, 100 or 120, more preferably the quenching reagent is an amino acid such as cysteine. Alternatively the groups may be reacted with a peptide or polysaccharide moiety capable of reacting with maleimide, for instance a peptide containing a cysteine residue. This is particularly appropriate where unreacted maleimide groups are present prior to the quenching step.

The detoxification step is suitable for use on bacterial toxins as described above. Preferably the bacterial toxin is from Streptococcus pneumoniae, most preferably the toxin is pneumolysin. The pneumolysin is a native or recombinant protein or a protein that has been genetically engineered to reduce its toxicity (as described above). Fusion proteins of toxins, preferably pneumolysin or fragments of toxins, preferably pneumolysin may be detoxified using the method of the invention.

Thus in a preferred embodiment, a toxin (such as pneumolysin) is detoxified with a cross-linking reagent which is preferably heterobifunctional having groups that are reactive with lysine and cysteine residues and is of a certain size, most preferably having the reactive groups spaced 10-20 Angstroms apart such that either or preferably both or the following occurs:
a) between 5 and 30, preferably around 12-14 amino acid residues of the toxin are modified by a cross-linker molecule covalently binding preferably to a lysine or arginine residue (preferably, as measured indirectly by an Ellman reaction), the other end having been quenched (preferably with cysteine) and/or;
b) a cysteine sidechain involved in the toxic activity of the toxin (preferably towards the C-terminus of the toxin) is cross-linked to another sidechain of the toxin (preferably to a lysine or arginine residue) which is preferably separated by more than 2, 5, 10, 20, 30 or 40 amino acids from the cysteine residue in the primary sequence of the toxin.

In a further preferred embodiment, a toxin (preferably pneumolysin) is detoxified with a monofunctional chemical compound which preferably reacts with amino acids containing a primary amine group, more preferably lysine, and is of a certain size, most preferably 10-100 Angstroms such that the toxin is covered with between 5 and 30, more preferably around 14 chemical compound bound to amino acid residues.

### Polysaccharide conjugates

A problem associated with the polysaccharide approach to vaccination, is the fact that polysaccharides *per* se are poor immunogens. To overcome this, polysaccharides may be conjugated to protein carriers, which provide bystander T-cell help. The process of the invention may advantageously contain a further step of conjugating the cytolysin, preferably pneumolysin to a bacterial polysaccharide, for instance a lipo-oligosaccharide or preferably a capsular polysaccharide.

A preferred conjugate comprises cytolysin, preferably pneumolysin obtained by the method of the invention conjugated to capsular polysaccharides derived from *Streptococcus pneumoniae*. The pneumococcal capsular polysaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F), or mixtures of two or more of said conjugates (4, 7, 9, 11, 13 or 23).

Cytolysin, preferably pneumolysin, purified by the process of the invention is also preferably conjugated to capsular polysaccharides or lipopolysaccharides or lipooligosaccharides from other strains of bacteria. Such polysaccharides can be isolated from, for example, *H. influenzae*, *H. influenzae* type B (Hib), *N. meningitidis,* Streptococci other than *S. pneumoniae* (e.g., Group B Streptococcus, *S. pyogenes*, etc.), Staphylococcus (e.g., *S. aureus, S. epidermidis*)*, E. coli,* Enterococcus (e.g., *E. faecalis* and *E*. *faecium*), etc. Preferably the polysaccharides are from H. influenzae type B (Hib), and/or N. meningitidis groups A, C, W135, and/or Y. Preferred lipooligosaccharides include those from *N. meningitidis* (immunotypes L2, L3, L4 and/or L6), *M. catarrhalis* and *H. influenzae*.

The polysaccharide may be linked to cytolysin, preferably pneumolysin, by any known method (for example, by Likhite, U.S. Patent 4,372,945 and by Armor *et al*., U.S. Patent 4,474,757). Preferably, CDAP conjugation is carried out (WO 95/08348). To enhance immunogenicity, the polysaccharides may be adjuvanted and/or lyophilised. The polysaccharides of the invention may be full size or sized post purification to smaller polysaccharides or oligosaccharides.

In a further aspect of the invention, the cytolysin, preferably pneumolysin may be combined with any of the polysaccharides or oligosaccharides described above, without conjugating the cytolysin to the polysaccharide or oligosaccharide to form an immunogenic composition or vaccine.

The process of the invention preferably comprises a further step of formulating cytolysin, preferably pneumolysin into a vaccine.

The term "polysaccharide" includes the full length form of polysaccharides isolated from the organism and encompasses capsular polysaccharides, lipopolysaccharides and lipooligosaccharides. It also encompasses sized polysaccharides and oligosaccharides.

The terms "comprising", "comprise" and "comprises" herein are intended by the inventors to be optionally substitutable with the terms "consisting of", "consist of" and "consists of", respectively, in every instance.

### Proteins and immunogenic compositions

Further described is cytolysin, preferably pneumolysin, purified by the method of the invention. This includes a pneumolysin-bacterial capsular polysaccharide conjugate made by the process of the invention.

Further described is an immunogenic composition comprising cytolysin, preferably pneumolysin or pneumolysin- bacterial capsular polysaccharide obtained by the process of the invention (as described above).

The immunogenic composition preferably further comprises one or more members of the pneumococcal choline binding protein family, preferably choline binding protein A or an immunogenic fragment thereof and/or one or more members of the poly histidine triad family (including fusion proteins thereof), preferably PhtA, PhtB, PhtD or PhtE or an immunogenic fragment thereof.

Concerning the Choline Binding Protein family (CbpX), members of this family were originally identified as pneumococcal proteins that could be purified by choline-affinity chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (CbpX)" is selected from the group consisting of Choline Binding Proteins as identified in WO97/41151, PbcA, SpsA, PspC, CbpA, CbpD, and CbpG. CbpA is disclosed in WO97/41151. CbpD and CbpG are disclosed in WO00/29434. PspC is disclosed in WO97/09994. PbcA is disclosed in WO98/21337.SpsA is a Choline binding protein disclosed in WO 98/39450. Preferably the Choline Binding Proteins are selected from the group consisting of CbpA, PbcA, SpsA and PspC.

Further described is CbpX truncates wherein "CbpX" is defined above and "truncates" refers to CbpX proteins lacking 50% or more of the Choline binding region (C). Preferably such proteins lack the entire choline binding region. More preferably, the such protein truncates lack (i) the choline binding region and (ii) a portion of the N-terminal half of the protein as well, yet retain at least one repeat region (R1 or R2). More preferably still, the truncate has 2 repeat regions (R1 and R2), more preferably the truncate retains the proline rich region (P). Examples of such truncates are NR1xR2 and R1xR2 as illustrated in WO99/51266 or WO99/51188 and NR1XR2P, however, other choline binding proteins lacking a similar choline binding region are also contemplated within the scope of this invention.

The LytX family is membrane associated proteins associated with cell lysis. The N-terminal domain comprises choline binding domain(s), however the LytX family does not have all the features found in the CbpA family noted above and thus the LytX family is considered distinct from the CbpX family. In contrast with the CbpX family, the C-terminal domain contains the catalytic domain of the LytX protein family. The family comprises LytA, B and C. With regards to the LytX family, LytA is disclosed in Ronda et al., Eur J Biochem, 164:621-624 (1987). LytB is disclosed in WO98/18930, and is also referred to as Sp46. LytC is also disclosed in WO 98/18930, and is also referred to as Sp91. A preferred member of that family is LytC.

Further described are LytX truncates wherein "LytX" is defined above and "truncates" refers to LytX proteins lacking 50% or more of the Choline binding region. Preferably such proteins lack the entire choline binding region. An example of such truncates can be found in the Examples section of this invention.

Further described are CbpX truncate-LytX truncate chimeric proteins (or fusions). Preferably this comprises NR1xR2 (or R1xR2, or NR1XR2P) of CbpX and the C-terminal portion (Cterm, i.e., lacking the choline binding domains) of LytX (e.g., LytCCterm or Sp91 Cterm). More preferably CbpX is selected from the group consisting of CbpA, PbcA, SpsA and PspC. More preferably still, it is CbpA. Preferably, LytX is LytC (also referred to as Sp91).

Further described is a PspA or PsaA, or truncates lacking the choline binding domain (C) optionally expressed as a fusion protein with LytX. Preferably, LytX is LytC.

The Pht (Poly Histidine Triad) family comprises proteins PhtA, PhtB, PhtD, and PhtE. The family is characterised by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. Preferred members of the family comprise PhtA, PhtB and PhtD. More preferably, it comprises PhtA or PhtD. It is understood, however, that the terms Pht A, B, D, and E refer to proteins having sequences disclosed in the citations below as well as naturally-occurring (and man-made) variants thereof that have a sequence homology that is at least 90% identical to the referenced proteins. Preferably it is at least 95% identical and most preferably it is 97% identical.
The imunogenic composition may incorporate fusion proteins of histidine triad proteins. These include fusion proteins with one histidine triad protein or fragment thereof linked to a second histidine triad protein or fragment thereof. Preferred fusion proteins contain i) PhtD or a fragment thereof linked to PhtE or a fragment thereof or ii) PhtB or a fragment thereof linked to PhtE or a fragment thereof.

With regards to the PhtX proteins, PhtA is disclosed in WO98/18930, and is also referred to Sp36. As noted above, it is a protein from the polyhistidine triad family and has the type II signal motif of LXXC.

PhtD is disclosed in WO 00/37105, and is also referred to Sp036D. As noted above, it also is a protein from the polyhistidine triad family and has the type II LXXC signal motif. PhtB is disclosed in WO00/37105, and is also referred to Sp036B. Another member of the PhtB family is the C3-Degrading Polypeptide, as disclosed in WO00/17370. This protein also is from the polyhistidine triad family and has the type II LXXC signal motif. A preferred immunologically functional equivalent is the protein Sp42 disclosed in WO 98/18930. A PhtB truncate (approximately 79kD) is disclosed in WO99/1567 which is also considered a member of the PhtX family.

PhtE is disclosed in WO00/30299 and is referred to as BVH-3.

In order to generate an immunogenic composition, capable of eliciting an immune response against more than one pathogen involved in otitis media, it is advantageous for immunogenic compositions of the invention to further comprise an antigen from one or more (2, 3, 4, 5, 6, ) of *S. pneumoniae*, non-typable Haemophilus influenzae, Moraxella catarrhalis, RSV, parainfluenza virus and /or influenza virus.

The present invention also describes combination vaccines which provide protection against a range of different pathogens. Many Paediatric vaccines are now given as a combination vaccine so as to reduce the number of injections a child has to receive. Thus for Paediatric vaccines other antigens from other pathogens may be formulated with the vaccines of the invention. For example the vaccines of the invention can be formulated with (or administered separately but at the same time) the well known 'trivalent' combination vaccine comprising Diphtheria toxoid (DT), tetanus toxoid (TT), and pertussis components [typically detoxified Pertussis toxoid (PT) and filamentous haemagglutinin (FHA) with optional pertactin (PRN) and/or agglutinin 1+2], for example the marketed vaccine INFANRIX-DTPa^{™} (SmithKlineBeecham Biologicals) which contains DT, TT, PT, FHA and PRN antigens, or with a whole cell pertussis component for example as marketed by SmithKlineBeecham Biologicals s.a., as Tritanrix^{™}. The combined vaccine may also comprise other antigen, such as Hepatitis B surface antigen (HBsAg), Polio virus antigens (for instance inactivated trivalent polio virus - IPV), *Moraxella catarrhalis* outer membrane proteins, non-typeable *Haemophilus influenzae* proteins, *N.meningitidis* B outer membrane proteins.

Examples of preferred *Moraxella catarrhalis* protein antigens which can be included in a combination vaccine (especially for the prevention of otitis media) are: OMP106 [WO 97/41731 (Antex) & WO96/34960 (PMC)]; OMP21; LbpA &/or LbpB [WO98/55606 (PMC)]; TbpA &/or TbpB [WO 97/13785 & WO97/32980 (PMC)]; CopB [Helminen ME, et al. (1993) Infect. Immun. 61:2003-2010]; UspA1 and/or UspA2 [WO93/03761 (University of Texas)]; OmpCD; HasR (PCT/EP99/03824); PilQ (PCT/EP99/03823); OMP85 (PCT/EP00/01468); lipo06 (GB 9917977.2); lipo10 (GB 9918208.1); lipo11 (GB 9918302.2); lipo18 (GB 9918038.2); P6 (PCT/EP99/03038); D15 (PCT/EP99/03822); OmplA1 (PCT/EP99/06781); Hly3 (PCT/EP99/03257); and OmpE. Examples of non-typeable *Haemophilus influenzae* antigens which can be included in a combination vaccine (especially for the prevention of otitis media) include: Fimbrin protein [(US 5766608 - Ohio State Research Foundation)] and fusions comprising peptides therefrom [eg LB1(f) peptide fusions; US 5843464 (OSU) or WO99/64067]; OMP26 [WO 97/01638 (Cortecs)]; P6 [EP 281673 (State University of New York)]; TbpA and/or TbpB; Hia; Hsf; Hin47; Hif; Hmw1; Hmw2; Hmw3; Hmw4; Hap; D15 (WO 94/12641); protein D (EP 594610); P2; and P5 (WO 94/26304).

Other combinations contemplated are the cytolysin, preferably pneumolysin of the invention in combination with viral antigens, for example, from influenza (attenuated, split, or subunit [e.g., surface glycoproteins neuraminidase (NA) and haemagglutinin (HA). See, e.g., Chaloupka 1. et al, Eur. Journal Clin. Microbiol. Infect. Dis. 1996, 15:121-127], RSV (e.g., F and G antigens or F/G fusions, see, eg, Schmidt A. C. et al, J Virol, May 2001, p4594 - 4603), parainfluenxa virus 3 (PIV3) (e.g., HN and F proteins, see Schmidt et al. supra), Varicella (e.g., attenuated, glycoproteins I-V, etc.), and any (or all) component(s) of MMR (measles, mumps, rubella).

### Vaccines

Further described is a vaccine comprising cytolysin, preferably pneumolysin or a pneumolysin-bacterial capsular polysaccharide conjugate, obtained by the process of the invention and a pharmaceutically acceptable excipient and optionally an adjuvant.

A vaccine may comprise the immunogenic compositions described above and a pharmaceutically acceptable excipient.

Vaccines are capable of generating a protective immune response against S. pneumoniae infection and/or otitis media.

A further embodiment of the invention includes a method of making a vaccine by taking a cytolysin, preferably pneumolysin, made by the process of the invention and formulating it as a vaccine with a pharmaceutically acceptable excipient and optionally with one or more of the further antigens described above.

Further described is a method of treatment or prevention of bacterial infection, preferably Streptococcus pneumoniae infection or otitis media comprising administration of the vaccine or immunogenic composition.

Further described is the use of the cytolysin, preferably pneumolysin and/or pneumolysin - bacterial capsular polysaccharide conjugate, either of which is obtained by a process of the invention, in the preparation of a vaccine for the treatment or prevention of bacterial infection, preferably Streptococcus pneumoniae infection or otitis media.

The vaccines are preferably adjuvanted. Suitable adjuvants include an aluminium salt such as aluminium hydroxide gel (alum) or aluminium phosphate, but may also be a salt of calcium, magnesium, iron or zinc, or may be an insoluble suspension of acylated tyrosine, or acylated sugars, cationically or anionically derivatised polysaccharides, or polyphosphazenes.
It is preferred that the adjuvant be selected to be a preferential inducer of a TH1 type of response. Such high levels of Th1-type cytokines tend to favour the induction of cell mediated immune responses to a given antigen, whilst high levels of Th2-type cytokines tend to favour the induction of humoral immune responses to the antigen.

It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of 11-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); and a combination of monophosphoryl lipid A, preferably 3-de-O-acytated monophosphoryl lipid A, together with either an aluminium salt (for instance aluminium phosphate or aluminium hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO96/33739.

A particularly potent adjuvant formulation involving QS21, 3D-MPL and tocopherol in an oil in water emulsion is described in WO95/17210, and is a preferred formulation.

Preferably the vaccine additionally comprises a saponin, more preferably QS21. The formulation may also comprise an oil in water emulsion and tocopherol (WO 95/17210).

The present invention also describes a method for producing a vaccine formulation comprising mixing a cytolysin together with a pharmaceutically acceptable excipient, such as 3D-MPL.

Unmethylated CpG containing oligonucleotides (WO 96/02555) are also preferential inducers of a TH1 response and are suitable for use in the present invention.

In a further aspect of the present invention there is described vaccine as herein described for use in medicine. Described is a method of preventing or ameliorating pneumonia in an elderly human (over 55 years old) comprising administering a safe and effective amount of a vaccine of the invention, and optionally a Th1 adjuvant, to said elderly patient.

In a further embodiment there is described a method of preventing or ameliorating otitis media in Infants (up to 24 months) or toddlers (typically 24 months to 5 years), comprising administering a safe and effective amount of a vaccine comprising a cytolysin, preferably pneumolysin, optionally with one or more of the further antigens described above and optionally a Th1 adjuvant, to said Infant or toddler.

The vaccine preparations may be used to protect or treat a mammal (preferably a human patient) susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Intranasal administration of vaccines for the treatment of pneumonia or otitis media is preferred (as nasopharyngeal carriage of pneumococci can be more effectively prevented, thus attenuating infection at its earliest stage). Although the vaccine may be administered as a single dose, components thereof may also be co-administered together at the same time or at different times (for instance if polysaccharides are present in a vaccine these could be administered separately at the same time or 1-2 weeks after the administration of the bacterial protein combination for optimal coordination of the immune responses with respect to each other). In addition to a single route of administration, 2 different routes of administration may be used. For example, viral antigens may be administered ID (intradermal), whilst bacterial proteins may be administered IM (intramuscular) or IN (intranasal). If polysaccharides are present, they may be administered IM (or ID) and bacterial proteins may be administered IN (or ID). In addition, the vaccines may be administered IM for priming doses and IN for booster doses.

The amount of conjugate antigen in each vaccine dose is selected as an amount which induces an immunoprotective response without significant, adverse side effects in typical vaccines. Such amount will vary depending upon which specific immunogen is employed and how it is presented. The content of protein antigens in the vaccine will typically be in the range 1-100µg, preferably 5-50µg, most typically in the range 5 - 25µg. If polysaccharides are included, generally it is expected that each dose will comprise 0.1-100 µg of polysaccharide, preferably 0.1-50 µg, more preferably 0.1-10 µg, of which 1 to 5 µg is the most preferable range.

Optimal amounts of components for a particular vaccine can be ascertained by standard studies involving observation of appropriate immune responses in subjects. Following an initial vaccination, subjects may receive one or several booster immunisations adequately spaced. Typically a vaccine will comprise antigen (proteins), an adjuvant, and excipients or a pharmaceutically acceptable carrier.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

Although the vaccines may be administered by any route, administration of the described vaccines into the skin (ID) forms one embodiment of the present invention. Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO99/34850 and EP 1092444, also the jet injection devices described for example in WO01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734; WO 98/28037).

When the vaccines are to be administered to the skin, or more specifically into the dermis, the vaccine is in a low liquid volume, particularly a volume of between about 0.05 ml and 0.2 ml.

The content of antigens in the skin or intradermal vaccines may be similar to conventional doses as found in intramuscular vaccines. Accordingly, the protein antigens present in the intradermal vaccines may in the range 1-100µg, preferably 5-50µg. Likewise, if present, the amount of polysaccharide conjugate antigen in each vaccine dose is generally expected to comprise 0.1-100 µg of polysaccharide, preferably 0.1-50 µg, preferably 0.1-10 µg, and may be between 1 and 5 µg. However, it is a feature of skin or intradermal vaccines that the formulations may be "low dose". Accordingly the protein antigens in "low dose" vaccines are preferably present in as little as 0.1 to 10µg, preferably 0.1 to 5 µg per dose; and if present the polysaccharide conjugate antigens may be present in the range of 0.01-1µg, and preferably between 0.01 to 0.5 µg of polysaccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

The immunogenic compositions and vaccines can be evaluated in various animal models or with human sera. As an illustration, the following animal models can be used to evaluate pneumococcal infection. C3H/HeJ Mice (6 to 8 week old) can be immunised s.c. with 15 µg protein adjuvanted with 50 µl CFA, followed 3-4 weeks later by boosting with 15 µg protein with IFA. For demonstrating passive and active protection from systemic infection, mice can be administered intraperitoneally with immune sera or proteins prior to challenge by intraperitoneal injection with 15 to 90 LD50 pneumococci on week 8-10. Additionally, proteins can be tested in a mouse nasopharynx colonization model by (Wu et al Microbial Pathogenesis 1997; 23:127-137).

In addition to mice, infant rats are susceptible to colonisation and infection by *S. pneumoniae*. In passive protective studies, administration of mouse immune sera (100 µl i.p. or 10 µl i.n.) can be done prior to challenge with intranasal administration of *S.pneumonia* (10 µl) in 2-5 day old infant rat pups. Colonisation can be determined by plating nasal washes (20-40 µl instilled, 10 µl withdrawn).

Favourable interactions between the protein (or protein and polysaccharide) components of the combination vaccine may be demonstrated by administering a dose of each protein (or protein and polysaccharide) in the vaccine which would be sub-protective in a monovalent vaccine. Increased protective efficacy of the combination vaccine compared to monovalent vaccines can be attributed to a favourable interaction between the components.

The invention is illustrated in the accompanying examples. The examples are carried out using standard techniques, which are well known and routine to those of skill in the art, except where otherwise described in detail. The examples are meant to illustrate, but not limit the invention.

### Examples

### Example 1 Purification of pneumolysin

Pneumolysin was expressed recombinantly in an *E*. *coli* culture. After 18 hours induction of the *E*. *coli* culture by increasing the temperature to 39.5 °C, the *E*. *coli* were pelletted by centrifugation at 17,000g for 1 hour. The pellet was resuspended in 25mM diethanolamine pH9.0 and the E. coli were mechanically broken using one pass at 500 PSI in a Rannie apparatus. 1% Sodium lauroyl sarcosinate (SLS) was added to the broken *E.coli* and the mixture was incubated for 1 hour at room temperature before centrifugation at 30,000g for 20 minutes so that cellular debris was pelleted. The supernatant was diluted 2.5 fold to end up in 20mM phosphate pH 7.0 containing 1M NaCl and 1% SLS and was then loaded onto a phenyl-sepharose HP column equilibrated in the same buffer (20mM phosphate pH 7.0 containing 1M NaCl and 1% SLS = equilibration buffer). The column was washed with 4 column volumes of equilibration buffer followed by 2 column volumes of 20mM phosphate buffer pH7.0 containing 0.5M NaCl and 1% SLS. Pneumolysin was eluted from the column by applying a low salt buffer containing 20mM phosphate buffer pH 7.0 containing 1% SLS. Fractions containing pneumolysin were identified using SDS-PAGE analysis, were pooled and the buffer was exchanged to 25mM diethanolamine pH 9.0 using diafiltration.

The pneumolysin was solubilised by denaturation by adding solid guanidine hydrochloride up to 6M final concentration and incubating for one hour. It was then diafiltered against 8M urea in 25mM diethanolamine pH9.0 containing 1 mM DTT. Pneumolysin was refolded by diafiltration against 20mM borate buffer pH9.0 containing 1 mM DTT. After renaturation, DTT was removed by diafiltration against 20mM borate buffer pH 9.0.

The purity of the pneumolysin achieved was analysed by running on an SDS-PAGE and staining with Coomassie brilliant blue. A separate gel was analysed by Western blotting using an antibody against *E*. *coli* to detect the level of *E.coli* proteins remaining in the purified pneumolysin preparation. The biological activity of the purified pneumolysin was assessed using an in vitro haemolysis assay.

### Results

As shown in figure 1, the method described above was able to produce a highly efficient purification of pneumolysin after a single chromatography step. The Coomassie blue stained gel in panel A shows that elution of the column with a low salt buffer containing no added sodium chloride was able to elute a 53kDa band corresponding to pneumolysin from the column in a highly purified form. The much fainter band of approximately 45kDa is also thought to be pneumolysin since this second band binds to anti-pneumolysin antibodies (results not show) and also fails to bind to the anti E. coli antibodies as shown in panel B. The Western blot of panel B is a highly sensitive method of detecting any contaminating proteins that remain in the purified pneumolysin. This method was able to detect very few contaminants and those present were at a low level that was below the detection level of Coomassie staining. The pneumolysin was therefore purified to a level of 98-100% purity.

The yield of the purification method was good with a typical run giving around 1900 mg of pneumolysin per litre of fermentation. Approximately 10% of the protein from the fermentation culture was recovered as purified pneumolysin.

The activity of the pneumolysin in a haemolysis assay was assessed after the pneumolysin had been treated with guanidinium hydrochoride/urea and had been refolded by removal of the denaturant. Haemolytic activity was detected in dilutions of the purified pneumolysin down to concentrations of 1.3 ng/ml showing that haemolytic activity had been re-established. This corresponds to between 500,000 and 1,000,000 Haemolytic units per mg of wild-type pneumolysin.

### Example 2 Purification of Pneumolysin omitting centrifugation

Pneumolysin was expressed recombinantly in an *E. coli* culture. After 18 hours induction of the *E*. *coli* culture by increasing the temperature to 39.5 °C, the culture was chilled to 20°C- and sodium lauroyl sarcosinate (SLS) was added to the culture to a final concentration of 1%. The culture was incubated in the presence of SLS for 30 minutes. Diethanolamine (DEA) was added to the culture to give a final concentration of 25mM and a pH of 9. The culture was mechanically broken using four passes at 1000 PSI in a Rannie apparatus. The culture was diluted to equivalent OD₆₀₀ of 60 and prefiltered by passing through a 0.65µm depth filter (for example a Millistock COHC filter). The filtered culture was diluted 2-fold into a buffer containing 1% SLS, 2M NaCl and having a pH of 7.0. The filtered culture was loaded onto a phenyl-sepharose HP column (Amersham) equilibrated in 20mM phosphate pH 7.0 containing 1M NaCl and 1% SLS (equilibration buffer). The column was washed with 4 column volumes of equilibration buffer followed by 2 column volumes of 20mM phosphate buffer pH7.0 containing 0.5M NaCl and 1% SLS. Pneumolysin was eluted from the column by applying a low salt (no NaCI) buffer containing 20mM phosphate buffer pH 7.0 containing 1% SLS. A single column step was able to produce pneumolysin of at least 90% purity. Where higher levels of purity are required, the pneumolysin may be passed over a gel filtration column. Fractions containing pneumolysin were identified using SDS-PAGE analysis, were pooled and the buffer was exchanged to 25mM diethanolamine pH 9.0 using diafiltration.

The pneumolysin was solubilised by denaturation by adding solid guanidine hydrochloride up to 6M final concentration and incubating for one hour. It was diafiltered against 8M urea in 25mM diethanolamine pH9.0 containing 1mM DTT. Pneumolysin was refolded by diafiltration against 20mM borate buffer pH9.0 containing 1mM DTT. The last diafiltration was carried out with a flow rate of 100ml/min for the first hour, 200ml/min for the second hour, 300ml/min for the third hour and 400ml/min for a further 2-3 hours. After renaturation, DTT was removed by diafiltration against 20mM borate buffer pH 9.0. Pure active pneumolysin was stored either at 4°C or frozen at -20°C or below.

The purity of the pneumolysin achieved was analysed by running on an SDS-PAGE and staining with Coomassie brilliant blue. The yield of pneumolysin using this method was approximately 1000mg/litre of fermentation. Analysis on an HR400 gel filtration column showed that 90-95% of the pneumolysin was monomeric. An in vitro haemolysis assay showed that haemolytic activity was retained, demonstrating that correct refolding of pneumolysin had been achieved.

### Example 3 - Detoxification of S. pneumoniae pneumolysin using GMBS

Purified pneumolysin was detoxified by modification of sulfhydryl and primary amine groups using the NHS ester-maleimide crosslinking reagent GMBS (N-(γ-maleimidobutyryloxy)succinimide ester). Pneumolysin at a concentration of 1mg/ml, was dialysed against 50mM borate buffer pH 9.0. The GMBS was initially dissolved in DMSO and was added to pneumolysin in at a 248-fold molar excess of GMBS. Treatment continued for one hour at room temperature. Excess GMBS and by-products were removed by dialysis against 100mM sodium phosphate pH 6.8. Further maleimide groups were quenched by reacting with 0.6mg/ml cysteine for two hours at room temperature. In order to remove excess cysteine, the sample was dialysed against 2mM sodium phosphate pH7.15.

### Example 4 - Detoxification of S. pneumoniae pneumolysin using GMBS

Purified pneumolysin was detoxified by modification of sulfhydryl and primary amine groups using the NHS ester-maleimide crosslinking reagent GMBS (N-(γ-maleimidobutyryloxy)succinimide ester). Pneumolysin at a concentration of 0.5 mg/ml, Pneumolysin at a concentration of 0.5 mg/ml, was put into a buffer of pH 7.5 by adding KH2PO4 and NaCl was added to 250mM. The GMBS was initially dissolved in DMSO and was added to pneumolysin in at a 248-fold molar excess of GMBS. Treatment continued for one hour at room temperature. Excess GMBS and by-products were removed by diafiltration using a 30kD membrane against 2mM sodium phosphate pH 7.15. The pH was then adjusted to 6.8 by addition of NaH2PO4. Further maleimide groups were quenched by reacting with 0.6mg/ml cysteine for two hours at room temperature. In order to remove excess cysteine, the sample was diafiltrated against 2mM sodium phosphate pH7.15 and concentrated to 1 mg/ml.

### Example 5 - Detoxification of S. Pneumoniae Pneumolysin using GMBS

Pneumolysin was firstly dialysed against 50 mM sodium phosphate buffer pH7.2. The heterobifunctional cross-linking reagent GMBS (N-[γ-maleimidobutyryloxy]succinimide ester, Pierce) was dissolved in DMSO at 10 mg/ml. An aliquot equal to the selected quantity of GMBS were added to 1 ml of pneumolysin (1 mg/ml). After 60 minutes at room temperature, the activated pneumolysin was purified either on a PD10 column (Amersham) either by gel filtration (Toyopearl HW-40, XK 16/40, elution with 100 mM sodium phosphate buffer pH 6.8) or either by dialysis in order to remove excess of reagent and GMBS by-products. The degree of derivatization of the PLY was measured by estimation of the maleimide functions using the Ellman's test (Aitken and Leurmonth p487-488 The protein protocols handbook Ed: J.M. Walker (1996) ). The maleimide functions were quenched by a solution of cysteine as a final step of the detoxification process (cysteine at 4 mg/ml (Merck), incubation during 60 minutes at room temperature). In order to remove excess cysteine, the sample was dialysed against 100 mM sodium phosphate buffer pH 6.8. The sample is then filtered through a sterilizing 0.22 µm membrane.

### Example 6 - Characterization of detoxified Pneumolysin

### Haemolytic activity

A hemolytic assay was used to assess the remaining toxicity of detoxified pneumolysin. Serial 2-fold dilutions of pneumolysin were incubated with sheep red blood cells. After centrifugation, the supernatant was transferred to immunoplates and released haemoglobin was measured using optical density reading at 405 nm. Results were expressed as ng/ml pneumolysin corresponding to the mid-point of the OD curve. The assay was repeated after incubating the detoxified pneumolysin at 37 °C for 7 days to monitor the reversibility of detoxification.

As shown in table 1, treatment with GMBS was able to substantially reduce the haemolytic activity of PLY with up to a 3,000 fold reduction in haemolytic activity being achieved. Higher molar ratios of GMBS/lysine were able to produce better removal of haemolytic activity with ratios of 4/1 and 5/1 being optimal in this experiment. This treatment was estimated to result in modification of about 14 lysine residues. Where fewer lysine residues were modified, the reduction in haemolytic activity was less.

### ELISA

The antigenicity of the detoxified pneumolysin was assessed by ELISA. The ELISA plates were coated with a guinea pig anti-pneumolysin antibody. Samples containing dilutions of pneumolysin were incubated in the plates for 1 hour at room temperature. After washing, the bound pneumolysin was detected using rabbit polyclonal antibodies against pneumolysin, conjugated to horseradish peroxidase. After washing the plates, a substrate reaction was used to assess the amount of pneumolysin bound to each well.

As shown in table 1, treatment with GMBS led to some loss of antigenicity as assessed by ELISA. However ELISA readings of approximately 66% of that given by untreated PLY could be achieved showing that many antibodies could still recognize the modified pneumolysin.

### SDS-PAGE-analysis

The detoxified pneumolysin proteins were run on an SDS-PAGE (Novex 4-20% polyacrylamide gel Invitrogen) and Coomassie brilliant blue was used to visualize the proteins. As shown on figure 2, treatment with GMBS led to a slight increase in the molecular weight of PLY from 53kDa to approximately 56kDa. This increase is due to the modification of multiple amino acid residues with GMBS. A small percentage of PLY is converted to multimeric forms as seen by the appearance of faint bands of molecular weight of approximately 110kDa and 170kDa, however, most of the PLY remains in an essentially monomeric form. Incubation of the PLY at 37°C for 7 days did not results in any substantial change in the appearance of the PLY on an SDS-PAGE showing that the modified PLY is not subject to degradation or subsequent covalently-linked multimer formation.

**Table 1: Trials of PLY detoxification by GMBS**

| **Trial** | **GMBS excess** | **Maleimide** | **Ratio** | | **Hemolytic titer** | |
|---|---|---|---|---|---|---|
| | **(GMBS/Lysine)** | **functions** | **ELISAILOWRY** | | **ng/m**l | |
| | | | **4°C** | **7D37°C** | **4°C** | **7D37°C** |
| | | | **%** | | | |
| | / | / | 95 | 56 | 1.7 | 4.2 |
| | 1/1 | 8 | 63 | 87 | 186 | 111 |
| | 1.5/1 | 8.5 | 69 | / | 48 | / |
| 1 | 2/1 | 9.4 | 76 | / | 309 | / |
| | 3/1 | 11.8 | 56 | / | 530 | / |
| | 4/1 | 13.5 | 66 | / | 6308 | / |
| | 5/1 | 14.2 | 67 | / | 4284 | / |
| 2 | 4/1 | 13.8 | 26.2 | 24.8 | NH | NH |
| | 8/1 | 17.6 | 23.9 | 38.0 | NH | NH |
| 3 | 4/1 | 11.3 | 89 | 46 | 1598 | 6309 |

| | | | | | | |
|---|---|---|---|---|---|---|
| Trials were realised on 1 mg of PLY (1 mg/ml) except for the last assay for which 3 mg were treated (PLY at 0.68 mg/ml). | | | | | | |

### Example 7 Reactogenicity evaluation of detoxified pneumolysin in rats

Groups of three OFA rats were immunised once by intramuscular (tibialis) inoculation with saline, pneumolysin or GMBS detoxified pneumolysin. Three days after immunisation, all the rats were killed and the tibialis were prepared for histological examination. The tibialis were fixed in formalin and cut into 2mm slices which were dehydrated and paraffin embedded. 7um sections were cut and stained using the Trichrome Masson method, before being examined microscopically.

Reactogenicity was evaluated using four criteria; degeneration/necrosis, endomysial inflammation, haemorrhage and aponeurosis inflammation. For each histological criterion, a score was attributed to each muscle of each group and a mean lesion score was then calculation for each group. A score of 0 = normal, 1 = minimal, 2 = slight, 3 = moderate, 4 = marked and 5 = severe.

### Results

The histology of sections was examined. The mean scores for degeneration/necrosis, endomysial inflammation, haemorrhage and aponeurosis inflammation are shown in Table 2.

**Table 2**

| Inoculation | Degeneration | Endomysial | Haemorrhage | Aponeurosis |
|---|---|---|---|---|
| | /Necrosis | inflammation | | inflammation |
| | | | | |
| NaCl | 0 | 0.5 | 0 | 0 |
| Ply | 3.6 | 3.8 | 3.0 | 1.4 |
| GMBS-Ply | 0.6 | 1.3 | 1.3 | 0.4 |

A comparison of histological scores for native and detoxified pneumolysin shows that GMBS is a particularly effective cross-linking reagent to use for the detoxification of pneumolysin, producing a large decrease in degeneration/necrosis, endomysial inflammation, haemorrhage and aponeurosis inflammation.

### Example 8 - Evaluation of toxicity of GMBS treated pneumolysin in mice

Groups of 20 OF1 mice were challenged intranasally with either native pneumolysin or GMBS-treated pneumolysin and the mice were monitored for the following 9 days.

As shown in Figure 3, challenge with 2µg of native pneumolysin led very quickly to the death of all the mice in that group. The pneumolysin produced lesions throughout the respiratory system which led to respiratory difficulties and death. In contrast, the GMBS treated pneumolysin had substantially reduced toxicity with all of the mice inoculated with 2µg, 5µg or 10µg of the GMBS treated pneumolysin surviving the challenge.

### Example 9 - protection studies using detoxified pneumolysin

Groups of 20 OF1 mice were immunised 3 times intramuscularly, on days 0, 14 and 28 with 5µg of pneumolysin and 50µg aluminium phosphate and 5µg MPL as adjuvant. Control mice were immunised with adjuvant alone. The pneumolysin was either untreated or detoxified using the GMBS treatment described above.

On day 42, the mice were given an intranasal, lethal challenge with 2µg of native pneumolysin. The survival of the mice over the following 9 days was monitored.

### Results

The lethal challenge model led to 90% mortality in control mice (Figure 4). Immunisation with GMBS detoxified pneumolysin produced very good protection with only 5% of mice dying during the following 9 days. This was comparable to protection given after inoculation with native pneumolysin, following which 10% of mice died.

### Example 10 Evaluation of detoxified pneumolysin in combination with PhtD in a mouse lethal challenge model

Groups of 20 OF1 mice were immunised intramuscularly with a) adjuvant alone or b) 1µg PhtD and adjuvant or c) 1µg PhtD and 5µg GMBS detoxified pneumolysin and adjuvant. The adjuvant used was composed of 50µg aluminium phosphate and 5µg MPL and immunisations took place on day 0 and day 14. The mice were challenged with an intranasal lethal dose of 5.10⁵ CFU of serotype 2 S. pneumoniae strain D39 and survival was monitored over the next 10 days.

### Results

As shown in Figure 5, challenge with strain D39 led to 75% lethality after 10 days in control mice. Immunisation with PhtD alone did not provide significant protection with 70% of mice in this group dying after 10 days (p=0.29). Immunisation with PhtD together with GMBS detoxified pneumolysin gave significantly better protection with lethality being reduced to 50% (p=0.04).

### Example 11 Detoxification of pneumolysin using formaldehyde

A stock of purified pneumolysin at a concentration of approximately 0.4 mg/ml was brought to 25 mM potassium phosphate, 50 mM lysine and 0.1% (w/v) formaldehyde. The pH was adjusted to 7.0 and the mixture was incubated for 21 days at 40°C. Unreacted formaldehyde, lysine and other low-molecular weight by-products were removed by diafiltration against sodium phosphate 2 mM pH 7.15.

### Example 12 Conjugation of GMBS-detoxified pneumolysin

GMBS treated pneumolysin (± 3 mg/ml in 20 mM borate buffer) was dialfiltrated against 2 mM phosphate buffer pH 6.8, 150 mM NaCl to reach a concentration between 15 and 20 mg/ml. The dialfiltration was performed on a Centramate membrane (0.09 m², 10 kDa cutoff).
Activation and coupling were carried out at 25°C. *S. pneumoniae* PS19F was diluted to 9 mg/ml in 2 M NaCl and the pH was adjusted to 6.0 by addition of 0.05N HCl. At time 0, a cyanodiaminopyridinium tetrafluoroborate (CDAP) solution (100 mg/ml in acetonitrile/water for injection: 50/50 (v/v)) was added manually in order to obtain a CDAP/PS19F ratio of 1.5 (w/w). After 1.5 minutes, the pH was raised to pH 9.0 by addition of 0.1M NaOH. GMBS treated pneumolysin (15 mg/ml) was then added in order to obtain a dPLY/PS ratio of 3. The solution was left for 240 min under pH regulation. The reaction was stopped by addition of 2M glycine (ratio Gly/PS ((w/w). The solution was left for 30 min before purification on Sephacryl S400HR. The conjugate has a dPLY/PS ratio of 2.62/1 (w/w).

A similar approach was also used to conjugate GMBS treated pneumolysin to PS8, PS12F and PS22F. The resulting conjugates have respectively a dPLY/PS ratio of 1.61, 1.45 and 1.36

### Example 13 Immunogenicity of S. pneumoniae capsule polysaccharides conjugated to GMBS-detoxified pneumolysin as a carrier protein

Groups of 40 female Balb/c mice were immunised by the intramuscular route at days 0, 14 and 28 with tetravalent polysaccharide (PS) formulations containing pneumococcal capsule PS 8, 12F, 19F and 22F (dose: 0.1 µg / PS).

Two formulations constituted of either plain PS or GMBS-detoxified pneumolysin conjugated PS were administered into mice. Both vaccine formulations were supplemented with an oil in water emulsion containing MPL and QS21.

Anti-PS ELISA IgG levels and opsono-phagocytosis titres were measured in sera collected at day 42 using the ELISA and opsonophagocytosis assays essentially as described in WO 02/22167. In the case of the opsonophagocytosis assay, serum samples were tested against *S. pneumoniae* serotypes 8, 12F, 19F and 22F. For the ELISA, the wells were coated with 10-40µg/ml of the required capsular polysaccharide.

### Results

A strong improvement of the IgG response was observed for all PS in mice immunised with PS conjugated to GMBS-detoxified pneumolysin, as compared to mice given plain PS (figure 6). The opsono-phagocytic activity (OPA) of anti-PS antibodies was also enhanced (figure 7).

## Claims

1. A process for purification of a bacterial cytolysin comprising the steps of :
a) growing a culture of cells expressing bacterial cytolysin;
b) mechanically breaking the culture of cells to form an extract;
c) prefiltering the extract;
d) binding soluble aggregated bacterial cytolysin contained in the extract in the presence of detergent to a hydrophobic interaction chromatography material under high salt (preferably 0.6-2M salt) conditions;
e) eluting bacterial cytolysin in the presence of detergent under low salt (preferably 0-0.2M salt) conditions.

2. The process of claim 1 further comprising the steps of:
f) removing detergent from the bacterial cytolysin
g) solubilising the bacterial cytolysin by addition of a denaturant;
h) removing the denaturant from the bacterial cytolysin.

3. The process of claim 1 or 2 wherein the bacterial cytolysin is pneumococcal pneumolysin.

4. The process of any one of claims 1-3 wherein step b) is carried out in the presence of detergent.

5. The process of claim 4 wherein the culture of cells is incubated with detergent before mechanically breaking the culture of cells in a preincubation step.

6. The process of claim 5 wherein the preincubation step lasts for over 10 minutes.

7. The process of any one of claims 1-6 wherein step b) and/or c) is carried out at a pH of 8-10.

8. The process of any one of claims 1-7 wherein step b) and/or c) is carried out at a salt concentration of 0-0.1M.

9. The process of any preceding claim wherein the prefiltering uses a filter size between 0.45 and 2.5µm.

10. The process of claims 1-9 wherein the same detergent is present in steps d) and e).

11. The process of claim 10 wherein the same detergent is present in steps b), d) and e).

12. The process of any preceding claim wherein the detergent is present in a concentration of between 0.1 and 2% (w/v).

13. The process of any preceding claim wherein no centrifugation step is present.

14. The process of any preceding claim wherein the hydrophobic interaction chromatography material used in step d) contains aromatic groups.

15. The process of claim 14 wherein the hydrophobic chromatography material is phenyl-sepharose.

16. The process of any preceding claim wherein the detergent present in the solution used in step b), c), d) and/or step e) is an aliphatic detergent.

17. The process of claim 16 wherein the aliphatic detergent is a detergent that is able to reduce the size of pneumolysin aggregates to render the pneumolysin aggregates soluble.

18. The process of any preceding claim wherein the detergent is sodium lauroyl sarcosinate.

19. The process of any preceding claim wherein the high salt conditions of step d) contains between 0.6M and 2M salt.

20. The process of any preceding claim wherein the solution used in step d) and/or e) contains a salt selected from the group consisting of sodium chloride, magnesium chloride, ammonium chloride, sodium sulphate, magnesium sulphate, ammonium sulphate, sodium phosphate, magnesium phosphate, ammonium phosphate.

21. The process of any preceding claim wherein step d) and/or step e) are carried out at a pH of between 6 and 8, preferably around 7.

22. The process of any preceding claim wherein the conditions used in step e) contain 0 - 0.1M salt.

23. The process of claim 22 wherein the conditions used in step e) contain 0-40mM salt.

24. The process of any preceding claim wherein step f) involves the removal of detergent by diafiltration or dialysis.

25. The process of claim 24 wherein the diafiltration/dialysis is against a low salt buffer of pH 8-10, preferably around pH 9.

26. The process of claims 2-25 wherein step g) involves denaturing the bacterial cytolysin by addition of a denaturant and step h) involves refolding the bacterial cytolysin by gradually removing the denaturant

27. The process of claims 2-26 wherein the denaturant used in step g) is guanidine hydrochloride.

28. The process of claim 27 wherein 5-8M guanidine hydrochloride is used.

29. The process of claims 26-28 wherein the bacterial cytolysin is contacted with 5-9M urea during step g).

30. The process of claim 29 wherein step g) involves contacting bacterial cytolysin with 5-8M guanidine hydrochloride followed by exchanging the guanidine hydrochloride for 5-9M urea.

31. The process of claims 26-30 wherein a reducing agent is present during at least part of steps g) and h).

32. The process of claim 31, wherein the reducing agent is 0.1-10mM DTT, preferably around 1mM DTT.

33. The process of claim 2-32 wherein in step h) bacterial cytolysin is refolded so that its haemolytic activity is restored to above 50% of that of the folded protein.

34. The process of claim 2-33 wherein step h) involves removal of the denaturant by diafiltration or dialysis.

35. The process of claim 34 wherein step h) involves removal of the denaturant in a linear fashion.

36. The process of claim 34 or 35 wherein step h) involves diafiltration at progressively higher flow rates.

37. The process of claim 34 or 35 wherein step h) involves dialysis against solutions containing progressively less denaturant.

38. The process of any one of claims 34-37 wherein diafiltration or dialysis is against a solution of pH 7-9.

39. The process of any preceding claim comprising a further step of detoxifying the bacterial cytolysin by chemical treatment.

40. The process of claim 39 wherein the chemical treatment involves use of a crosslinking agent.

41. The process of claim 40 wherein the crosslinking reagent contains one or more chemicals selected from the group consisting of: formaldehyde, glutaraldehyde, N-hydroxysuccinomido esters and GMBS.

42. The process of any preceding claim comprising a further step of conjugating the bacterial cytolysin to a bacterial polysaccharide.

43. The process of any preceding claim comprising a further step of formulating bacterial cytolysin into a vaccine composition with a pharmaceutically acceptable excipient.

44. The process of any preceding claim, comprising a further step of formulating bacterial cytolysin with one or more of: choline binding protein A or an immunogenic fragment thereof, or PhtA, PhtB, PhtD or PhtE or an immunogenic fragment thereof, or an antigen from non typeable *Haemophilus influenzae* (NtHi), or an antigen from *Moraxella catarrhalis* or an antigen from RSV, or an antigen from parainfluenza virus, or an antigen from influenza virus.

45. The process of any preceding claim, comprising a further step of formulating bacterial cytolysin with an adjuvant selected from a group consisting of: an aluminium salt such as aluminium hydroxide gel (alum) or aluminium phosphate, a salt of calcium, magnesium, iron or zinc, an insoluble suspension of acylated sugars, cationically or anionically derivatised polysaccharides, polyphosphazenes, MPL or a derivative thereof, 3D-MPL, saponin, QS21 and unmethylated CpG containing oligonucleotide.

## Patentansprüche

1. Verfahren zur Reinigung eines bakteriellen Cytolysins, umfassen die folgenden Schritte:
a) Züchten einer Kultur von Zellen, die bakterielles Cytolysin exprimieren;
b) mechanisches Aufbrechen der Kultur von Zellen, um einen Extrakt zu bilden;
c) Vorfiltrieren des Extrakts;
d) Binden von löslichen aggregierten bakteriellen Cytolysin, enthaltend in dem Extrakt, in Gegenwart eines Detergens an ein hydrophobes Wechselwirkungs-Chromatographiemateriel unter Hochsalzbedingungen (vorzugsweise 0,6-2 M Salz);
e) Eluieren des bakterielle Cytolysin in Gegenwart eines Detergens unter Niedrigsalzbedingungen (vorzugsweise 0-0,2 M Salz).

2. Verfahren gemäß Anspruch 1, ferner umfassend die folgenden Schritte:
f) Entfernung des Detergens vom bakteriellen Cytolysin;
g) Löslichmachen des bakteriellen Cytolysins durch Zugabe eines Denaturierungsmittels;
h) Entfernung des Denaturierungsmittels vom bakteriellen Cytolysin;

3. Verfahren gemäß Anspruch 1 oder 2, wobei das bakterielle Cytolysin Pneumokokken-Pneumolysin ist.

4. Verfahren gemäß einem der Ansprüche 1 bis 3, wobei Schritt b) in Gegenwart eines Detergens durchgeführt wird.

5. Verfahren gemäß Anspruch 4, wobei die Kultur von Zellen in einem Vorinkubationsschritt mit Detergens inkubiert wird, bevor die Kultur von Zellen mechanisch aufgebrochen wird.

6. Verfahren gemäß Anspruch 5, wobei der Vorinkubationsschritt mehr als 10 Minuten dauert.

7. Verfahren gemäß einem der Ansprüche 1 bis 6, wobei Schritt b) und/oder c) bei einem pH von 8 bis 10 durchgeführt wird.

8. Verfahren gemäß einem der Ansprüche 1 bis 7, wobei Schritt b) und/oder c) bei einer Salzkonzentration von 0 bis 0,1 M durchgeführt wird.

9. Verfahren gemäß einem der vorangegangenen Ansprüche, wobei das Vorfiltrieren eine Filtergröße zwischen 0,45 und 2,5 µm verwendet.

10. Verfahren gemäß den Ansprüchen 1 bis 9, wobei dasselbe Detergens in den Schritten d) und e) vorliegt.

11. Verfahren gemäß Anspruch 10, wobei dasselbe Detergens in den Schritten b), d) und e) vorliegt.

12. Verfahren gemäß einem der vorangegangenen Ansprüche, wobei das Detergens in einer Konzentration von zwischen 0,1 und 2 % (G/V) vorliegt.

13. Verfahren gemäß einem der vorangegangenen Ansprüche, wobei kein Zentrifugationsschritt vorliegt.

14. Verfahren gemäß einem der vorangegangenen Ansprüche, wobei das hydrophobe Wechselwirkungs-Chromatographiematerial, das in Schritt d) verwendet wird, aromatische Gruppen enthält.

15. Verfahren gemäß Anspruch 14, wobei das hydrophobe Chromatographie-Material Phenyl-Sepharose ist.

16. Verfahren gemäß einem der vorangegangenen Ansprüche, wobei das in der im Schritt b), c), d) und/oder Schritt e) verwendeten Lösung vorliegenden Detergens ein aliphatisches Detergens ist.

17. Verfahren gemäß Anspruch 16, wobei das aliphatische Detergens ein Detergens ist, das in der Lage ist, die Größe der Pneumolysin-Aggregate zu reduzieren, um die Pneumolysin-Aggregate löslich zu machen.

18. Verfahren gemäß einem der vorangegangenen Ansprüche, wobei das Detergens Natriumlauroylsarcosinat ist.

19. Verfahren gemäß einem der vorangegangenen Ansprüche, wobei die Hochsalzbedingungen gemäß Schritt d) zwischen 0,6 M und 2 M Salz enthalten.

20. Verfahren gemäß einem der vorangegangenen Ansprüche, wobei die in Schritt d) und/oder e) verwendete Lösung ein Salz enthält, das aus der Gruppe ausgewählt ist, die aus Natriumchlorid, Magnesiumchlorid, Ammoniumchlorid, Natriumsulfat, Magnesiumsulfat, Ammoniumsulfat, Natriumphosphat, Magnesiumphosphat und Ammoniumphosphat besteht.

21. Verfahren gemäß einem der vorangegangenen Ansprüche, wobei Schritt d) und/oder Schritt e) bei einem pH von zwischen 6 und 8, vorzugsweise bei ungefähr 7, durchgeführt werden.

22. Verfahren gemäß einem der vorangegangenen Ansprüche, wobei die in Schritt e) verwendeten Bedingungen 0 bis 0,1 M Salz enthalten.

23. Verfahren gemäß Anspruch 22, wobei die in Schritt e) verwendeten Bedingungen 0 bis 40 mM Salz enthalten.

24. Verfahren gemäß einem der vorangegangenen Ansprüche, wobei Schritt f) das Entfernen des Detergens durch Diafiltration oder Dialyse involviert.

25. Verfahren gemäß Anspruch 24, wobei die Diafiltration/Dialyse gegen einen Niedrig-Salzpuffer von pH 8 bis 10, vorzugsweise ungefähr pH 9, erfolgt.

26. Verfahren gemäß den Ansprüchen 2 bis 25, wobei Schritt g) das Denaturieren des bakteriellen Cytolysins durch Zugabe eines Deriaturierungsmittels und Schritt h) das neuerliche Falten des bakteriellen Cytolysins durch graduelle Entfernung des Denaturierungsmittels involviert.

27. Verfahren gemäß den Ansprüchen 2 bis 26, wobei das in Schritt g) verwendete Denaturierungsmittel Guanidinhydrochlorid ist.

28. Verfahren gemäß Anspruch 27, wobei 5 bis 8 M Guanidinhydrochlorid verwendet wird.

29. Verfahren gemäß den Ansprüchen 26 bis 28, wobei das bakterielle Cytolysin mit 5 bis 9 M Harnstoff während Schritt g) kontaktiert wird.

30. Verfahren gemäß Anspruch 29, wobei Schritt g) das Kontaktieren des bakteriellen Cytolysins mit 5 bis 8 M Guanidinhydrochlorid, gefolgt von einem Austausch des Guanidinhydrochlorids gegen 5 bis 9 M Harnstoff involviert.

31. Verfahren gemäß den Ansprüchen 26 bis 30, wobei ein Reduktionsmittel während mindestens einem Teil der Schritte g) und h) vorliegt.

32. Verfahren gemäß Anspruch 31, wobei das Reduktionsmittel 0,1 bis 10 mM DTT, vorzugsweise ungefähr 1 mM DTT, ist.

33. Verfahren gemäß den Ansprüchen 2 bis 32, wobei in Schritt h) das bakterielle Cytolysin so zurückgefaltet wird, daß seine hämolytische Aktivität bei über 50 % von derjenigen des richtig gefalteten Proteins wieder hergestellt wird.

34. Verfahren gemäß den Ansprüche 2 bis 33, wobei Schritt h) das Entfernen des Denaturierungsmittels durch Diafiltration oder Dialyse involviert.

35. Verfahren gemäß Anspruch 34, wobei Schritt h) das Entfernen des Denaturierungsmittels in einer linearen Weise involviert.

36. Verfahren gemäß Anspruch 34 oder 35, wobei Schritt h) eine Diafiltration bei progressiv höheren Flußraten involviert.

37. Verfahren gemäß Anspruch 34 oder 35, wobei Schritt h) eine Dialyse gegen Lösungen involviert, die progressiv weniger Denaturierungsmittel enthalten.

38. Verfahren gemäß einem der Ansprüche 34 bis 37, wobei die Diafiltration oder Dialyse gegen eine Lösung von pH 7 bis 9 erfolgt.

39. Verfahren gemäß einem der vorangegangenen Ansprüche, umfassend einen weiteren Schritt der Entgiftung des bakteriellen Cytolysins durch chemische Behandlung.

40. Verfahren gemäß Anspruch 39, wobei die chemische Behandlung die Verwendung eines Vernetzungsmittels involviert.

41. Verfahren gemäß Anspruch 40, wobei das Vernetzungsreagens ein oder mehrere Chemikalien enthält, die aus der Gruppe ausgewählt sind, bestehend aus: Formaldehyd, Glutaraldehyd, N-Hydroxysuccinomidoestern und GMBS.

42. Verfahren gemäß einem der vorangegangenen Ansprüche, umfassend einen weiteren Schritt der Konjugation des bakteriellen Cytolysins an ein bakterielles Polysaccharid.

43. Verfahren gemäß einem der vorangegangenen Ansprüche, umfassend einen weiteren Schritt der Formulierung des bakteriellen Cytolysins in eine Impfstoffzusammensetzung mit einem pharmazeutisch annehmbaren Exzipienten.

44. Verfahren gemäß einem der vorangegangenen Ansprüche, umfassend einen weiteren Schritt der Formulierung des bakteriellen Cytolysins mit einem oder mehreren von: Cholin-Bindungsprotein A oder einem immunogenen Fragment davon, oder PhtA, PhtB, PhtD oder PhtE oder einem immunogenen Fragment davon, oder einem Antigen von nicht-typisierbarem Haemophilus influenzae (NtHi), oder einem Antigen von Moraxella catarrhalis oder einem Antigen von RSV oder einem Antigen von ParainfluenzaVirus oder einem Antigen von Influenza-Virus.

45. Verfahren gemäß einem der vorangegangenen Ansprüche, umfassend einen weiteren Schritt der Formulierung des bakteriellen Cytolysins mit einem Adjuvans, das aus der Gruppe ausgewählt ist, die aus folgendem besteht: einem Aluminiumsalz wie Aluminiumhydroxid-Gel (Alaun) oder Aluminiumphosphat, einem Salz von Calcium, Magnesium, Eisen oder Zink, einer unlöslichen Suspension von acylierten Zuckern, kationisch oder anionisch derivatisierten Polysacchariden, Polyphosphazenen, MPL oder einem Derivat davon, 3D-MPL, Saponin, QS21 und nicht-methyliertes CpG-enthaltendes Oligonukleotid.

## Revendications

1. Procédé de purification d'une cytolysine bactérienne comprenant les étapes consistant à :
a) mettre en culture des cellules exprimant la cytolysine bactérienne ;
b) fractionner, de façon mécanique, les cellules en culture pour obtenir un extrait ;
c) préfiltrer l'extrait ;
d) lier la cytolysine bactérienne agrégée soluble contenue dans l'extrait en présence de détergent à un matériau de chromatographie d'interactions hydrophobes dans des conditions de salinité élevée (de préférence concentration en sel de 0,6 à 2 M) ;
e) éluer la cytolysine bactérienne en présence de détergent dans des conditions de faible salinité (de préférence concentration en sel de 0 à 0,2 M).

2. Procédé selon la revendication 1 comprenant les étapes consistant à :
f) éliminer le détergent de la cytolysine bactérienne ;
g) solubiliser la cytolysine bactérienne par addition d'un dénaturant ;
h) éliminer le dénaturant de la cytolysine bactérienne.

3. Procédé selon la revendication 1 ou 2, dans lequel la cytolysine bactérienne est une pneumolysine pneumococcique.

4. Procédé selon l'une quelconque des revendications 1 à 3, dans lequel l'étape b) est mise en oeuvre en présence de détergent.

5. Procédé selon la revendication 4, dans lequel les cellules en culture sont incubées avec du détergent avant leur fractionnement mécanique au cours d'une étape de pré-incubation.

6. Procédé selon la revendication 5, dans lequel l'étape de pré-incubation dure plus de 10 minutes.

7. Procédé selon l'une quelconque des revendications 1 à 6, dans lequel l'étape b) et/ou c) sont mises en oeuvre à un pH de 8 à 10.

8. Procédé selon l'une quelconque des revendications 1 à 7, dans lequel l'étape b) et/ou c) sont mises en oeuvre à une concentration en sel de 0 à 0,1 M.

9. Procédé selon l'une quelconque des revendications précédentes, dans lequel lors de la préfiltration, on utilise un filtre d'une taille comprise entre 0,45 et 2,5 µm.

10. Procédé selon les revendications 1 à 9, dans lequel le même détergent est présent aux étapes d) et e).

11. Procédé selon la revendication 10, dans lequel le même détergent est présent aux étapes b), d) et e).

12. Procédé selon l'une quelconque des revendications précédentes, dans lequel le détergent est présent à une concentration comprise entre 0,1 et 2 % (poids/volume).

13. Procédé selon l'une quelconque des revendications précédentes, ne comportant pas d'étape de centrifugation.

14. Procédé selon l'une quelconque des revendications précédentes, dans lequel le matériau de chromatographie d'interactions hydrophobes utilisé à l'étape d) contient des groupes aromatiques.

15. Procédé selon la revendication 14, dans lequel le matériau de chromatographie d'interactions hydrophobes est du phényl-sépharose.

16. Procédé selon l'une quelconque des revendications précédentes, dans lequel le détergent présent dans la solution utilisée aux étapes b), c), d) et/ou e) est un détergent aliphatique.

17. Procédé selon la revendication 16, dans lequel le détergent aliphatique est un détergent capable de réduire la taille des agrégats de pneumolysine afin de rendre ceux-ci solubles.

18. Procédé selon l'une quelconque des revendications précédentes, dans lequel le détergent est du lauroyl sarcosinate de sodium.

19. Procédé selon l'une quelconque des revendications précédentes, dans lequel les conditions de salinité élevée de l'étape d) correspondent à une concentration en sel comprise entre 0,6 et 2 M.

20. Procédé selon l'une quelconque des revendications précédentes, dans lequel la solution utilisée à l'étape d) et/ou e) contient un sel choisi dans le groupe constitué du chlorure de sodium, du chlorure de magnésium, du chlorure d'ammonium, du sulfate de sodium, du sulfate de magnésium, du sulfate d'ammonium, du phosphate de sodium, du phosphate de magnésium, du phosphate d'ammonium.

21. Procédé selon l'une quelconque des revendications précédentes, dans lequel l'étape d) et/ou l'étape e) sont mises en oeuvre à un pH compris entre 6 et 8 et, de préférence, de l'ordre de 7.

22. Procédé selon l'une quelconque des revendications précédentes, dans lequel les conditions de l'étape e) impliquent le recours à une concentration en sel de 0 à 0,1 M.

23. Procédé selon la revendication 22, dans lequel les conditions de l'étape e) impliquent le recours à une concentration en sel de 0 à 40 mM.

24. Procédé selon l'une quelconque des revendications précédentes, dans lequel l'étape f) implique l'élimination du détergent par diafiltration ou dialyse.

25. Procédé selon la revendication 24, dans lequel la diafiltration/dialyse se fait contre un tampon à faible teneur en sel ayant un pH de 8 à 10 et, de préférence, de l'ordre de 9.

26. Procédé selon les revendications 2 à 25, dans lequel l'étape g) implique la dénaturation de la cytolysine bactérienne par addition d'un dénaturant et l'étape h) implique le repliement de la cytolysine bactérienne par élimination progressive du dénaturant.

27. Procédé selon les revendications 2 à 26, dans lequel le dénaturant utilisé à l'étape g) est de l'hydrochlorure de guanidine.

28. Procédé selon la revendication 27, dans lequel on utilise de l'hydrochlorure de guanidine 5 à 8 M.

29. Procédé selon les revendications 26 à 28, dans lequel la cytolysine bactérienne est mise en contact avec de l'urée 5 à 9 M au cours de l'étape g).

30. Procédé selon la revendication 29, dans lequel l'étape g) implique la mise en contact de la cytolysine bactérienne avec de l'hydrochlorure de guanidine 5 à 8 M, cela étant suivi de l'échange de l'hydrochlorure de guanidine par de l'urée 5 à 9 M.

31. Procédé selon les revendications 26 à 30, dans lequel un agent réducteur est présent durant au moins une partie des étapes g) et h).

32. Procédé selon la revendication 31, dans lequel l'agent réducteur est constitué de 0,1 à 10 mM de DTT et, de préférence, d'environ 1 mM de DTT.

33. Procédé selon les revendications 2 à 32, dans lequel, au cours de l'étape h), la cytolysine bactérienne est repliée de façon à ce que son activité hémolytique soit restaurée et puisse atteindre plus de 50 % de celle de la protéine pliée.

34. Procédé selon les revendications 2 à 33, dans lequel l'étape h) implique l'élimination du dénaturant par diafiltration ou dialyse.

35. Procédé selon la revendication 34, dans lequel l'étape h) implique une élimination linéaire du dénaturant.

36. Procédé selon la revendication 34 ou 35, dans lequel l'étape h) implique une diafiltration à des débits de plus en plus élevés.

37. Procédé selon la revendication 34 ou 35, dans lequel l'étape h) implique une dialyse contre des solutions contenant de moins en moins de dénaturant.

38. Procédé selon l'une quelconque des revendications 34 à 37, dans lequel la diafiltration ou la dialyse se fait contre une solution présentant un pH de 7 à 9.

39. Procédé selon l'une quelconque des revendications précédentes, comprenant une étape supplémentaire consistant à détoxifier la cytolysine bactérienne par un traitement chimique.

40. Procédé selon la revendication 39, dans lequel le traitement chimique implique l'utilisation d'un agent de réticulation.

41. Procédé selon la revendication 40, dans lequel le réactif de réticulation contient au moins un composé chimique choisi dans le groupe constitué du formaldéhyde, du glutaraldéhyde, des esters N-hydroxysuccinimido et du GMBS.

42. Procédé selon l'une quelconque des revendications précédentes comprenant une étape supplémentaire consistant à conjuguer la cytolysine bactérienne avec un polysaccharide bactérien.

43. Procédé selon l'une quelconque des revendications précédentes comprenant une étape supplémentaire consistant à faire figurer la cytolysine bactérienne dans la formule d'une composition vaccinale avec un excipient acceptable d'un point de vue pharmaceutique.

44. Procédé selon l'une quelconque des revendications précédentes, comprenant une étape supplémentaire consistant à associer, dans la formule, la cytolysine bactérienne avec au moins l'une des substances suivantes : la protéine A liant la choline ou un fragment immunogène de celle-ci, ou PhtA, PhtB, PhtD ou PhtE ou un fragment immunogène de ceux-ci, ou un antigène de l'*Haemophilus influenzae* non typable (NtHi), ou un antigène de *Moraxella catarrhalis* ou un antigène du VRS, ou un antigène du virus parainfluenza, ou enfin un antigène du virus de la grippe.

45. Procédé selon l'une quelconque des revendications précédentes, comprenant une étape supplémentaire consistant à associer, dans la formule, la cytolysine bactérienne avec un adjuvant choisi dans un groupe constitué d'un sel d'aluminium, tel que le gel d'hydroxyde d'aluminium (alun) ou le phosphate d'aluminium, un sel de calcium, de magnésium, de fer ou de zinc, une suspension insoluble de sucres acylés, des polysaccharides cationiquement ou anioniquement dérivatisés, des polyphosphazènes, du MPL ou un dérivé de celui-ci, du 3D-MPL, de la saponine, du QS21 et des oligonucléotides contenant des motifs CpG non méthylés.
